(19)

Europäisches
Patentamt
European
Patent Office
Office européen
des brevets

(11)  EP 3 506 826 B1

(12)  **EUROPEAN PATENT SPECIFICATION**

(45) Date of publication and mention
of the grant of the patent:
**13.08.2025 Bulletin 2025/33**

(21) Application number: **17757817.6**

(22) Date of filing: **15.08.2017**

(51) International Patent Classification (IPC):
**A61B 5/08** (2006.01)   **A61B 5/091** (2006.01)

(52) Cooperative Patent Classification (CPC):
**A61B 5/091; A61B 5/082; G16H 20/40;**
**G16H 50/50;** A61B 5/0075; A61B 5/097

(86) International application number:
**PCT/GB2017/052397**

(87) International publication number:
**WO 2018/042152 (08.03.2018 Gazette 2018/10)**

(54) **METHOD AND APPARATUS FOR QUANTIFYING LUNG FUNCTION**

VERFAHREN UND VORRICHTUNG ZUR QUANTIFIZIERUNG EINER LUNGENFUNKTION

PROCÉDÉ ET APPAREIL DE QUANTIFICATION DE LA FONCTION PULMONAIRE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **01.09.2016 GB 201614842**
**08.09.2016 GB 201615277**

(43) Date of publication of application:
**10.07.2019 Bulletin 2019/28**

(73) Proprietor: **Oxford University Innovation Limited**
**Oxford, Oxfordshire OX2 0JB (GB)**

(72) Inventors:
• **ROBBINS, Peter Alistair**
**Oxford**
**Oxfordshire OX1 3PT (GB)**
• **HANCOCK, Gus**
**Oxford**
**Oxfordshire OX1 3QZ (GB)**
• **RITCHIE, Grant**
**Oxford**
**Oxfordshire OX1 3QZ (GB)**
• **MOUNTAIN, James**
**Oxford**
**Oxfordshire OX1 3PT (GB)**
• **O'NEILL, David**
**Oxford**
**Oxfordshire OX1 3PT (GB)**

• **WHITELEY, Jonathan**
**Oxford**
**Oxfordshire OX1 3QD (GB)**
• **CIAFFONI, Luca**
**Oxford**
**Oxfordshire OX1 3QZ (GB)**
• **COUPER, John**
**Reading**
**Berkshire RG7 7RL (GB)**

(74) Representative: **J A Kemp LLP**
**80 Turnmill Street**
**London EC1M 5QU (GB)**

(56) References cited:
**EP-B- 1 065 973      US-A1- 2015 272 475**

• **K. C. BECK ET AL: "Ventilation-perfusion**
**distribution in normal subjects", JOURNAL OF**
**APPLIED PHYSIOLOGY., vol. 113, no. 6, 15**
**September 2012 (2012-09-15), US, pages 872 -**
**877, XP055420410, ISSN: 8750-7587, DOI:**
**10.1152/japplphysiol.00163.2012**
• **L. CIAFFONI ET AL: "In-airway molecular flow**
**sensing: A new technology for continuous,**
**noninvasive monitoring of oxygen consumption**
**in critical care", SCIENCE ADVANCES, vol. 2, no.**
**8, 10 August 2016 (2016-08-10), pages e1600560 -**
**e1600560, XP055420334, DOI: 10.1126/**
**sciadv.1600560**

- STEVEN TREPPO ET AL: "Contributions of pulmonary perfusion and ventilation to heterogeneity in V&dot;a/Q&dot;measured by PET", JOURNAL OF APPLIED PHYSIOLOGY., vol. 82, no. 4, 1 April 1997 (1997-04-01), US, pages 1163 - 1176, XP055421949, ISSN: 8750-7587

**Description**

The present invention relates to a method and apparatus for quantifying lung function.

The quantification of lung function by pulmonary function tests (PFT) is important in the assessment of airways disease such as chronic obstructive pulmonary disease (COPD), asthma and cystic fibrosis. The quantification of lung function helps with assessing the degree and progress of disease and can be used by pharmaceutical companies to identify groups of patients for testing of different therapies and to assess the effectiveness of potential therapies.

The current gold standard pulmonary function test is the forced expiratory volume (FEV1) which is a measurement of the maximal amount of air that a patient can forcefully exhale in one second. This is the main pulmonary function test used to test for, and stage, COPD because it is inexpensive, quick and relatively simple to measure, and it integrates a number of features of COPD into a single measured variable. However, FEV1 does not detect early airway disease and is an extremely insensitive marker for changes in airway function. Its lack of sensitivity means that when used in Phase II clinical trials to assess the effectiveness of potential treatments, large numbers of patients must be recruited and a very high degree of patient cooperation is required.

Further, COPD is very heterogeneous. There are a number of distinct pathological changes that occur in the lung, their relative contributions to disease differ in different patients, and the degree of pathology varies significantly between different sections of the lung. These differences are reflected in the considerable variations in gas exchange within the diseased lung. The rate of decline in lung function also varies considerably between patients. Because COPD is so heterogeneous, there is considerable interest in profiling patients with COPD with a view to identifying sub-groups that may be amenable to different treatments.

Other techniques for lung function testing apart from spirometry, of which FEV1 is an example, are limited to measuring flows and volumes of particular types (addressing obstructive and restrictive patterns of lung disease), measuring carbon monoxide diffusing capacity (which measures the diffusing capacity of the lung) and measuring arterial blood gas. Other niche developments such as oscillometry or wash-out tests to give a lung clearance index have to date provided only limited information about the lung. For example, multiple-breath and single-breath inert gas wash-out tests (MBW and SBW, respectively) assess the efficiency of ventilation distribution in the lung. The paper "Ventilation-Perfusion Distribution in Normal Subjects" by Kenneth C Beck et al. (J. Appl. Physiol. 113: 872-877, 2012) on which the precharacterizing portion of claim 1 is based discusses a wash-in experiment for examining the ventilation-perfusion distribution of the lung in normal subjects. The paper "Slow and Fast Lung Compartments in Cystic Fibrosis Measured by Nitrogen Multiple-Breath Wash-out" by Gustafsson et al. (J. Appl. Physiol. 117: 720-729, 2014) discusses a nitrogen MBW test for assessing the influence of airway disease on the uniformity of ventilation distribution in the lungs. The ERS/ATS consensus statement for inert gas wash-out measurement using multiple- and single-breath tests by P D Robinson et al. (European Respiratory Journal 41: 507-522, 2013) notes that inert gas wash-out tests using the single- or multiple- breath wash-out technique have been known for several decades and in giving measures of ventilation distribution inhomogeneity offer complimentary information to standard lung function tests and improved sensitivity in the detection of early lung damage. However it also notes that there are significant limitations and difficulties in current techniques. US-A1-2015/0272475 and the paper "In-airway molecular flow sensing: A new technology for continuous, non-invasive monitoring of oxygen consumption in critical care" by Luca Ciaffoni et al. Science Advances, vol. 2, no. 8, 10 August 2016, pp e1600560-e1600560, disclose spectrometers for breath analysis.

According to the present invention there is provided a method and apparatus for quantifying lung function of a subject as defined by the appended claims.

By using breath sampling within each breath across multiple breaths the pattern by which respiratory gas comes out during a single breath can be joined with information contained within the pattern by which respiratory gas comes out during multiple breaths (with different volumes, durations and inspirates) in determining the parameters of the model. This provides much more sensitive and discriminatory measurements of lung function than is currently available.

By steady breathing is meant the supply of a constant, stable composition of oxygen and carbon dioxide in the inspiratory gas (such as air) which will result in stable respiration and a relatively stable composition for oxygen and carbon dioxide in mixed expired gas (once the subject has settled down). Optionally a subject may need to be allowed to breathe steadily for 2 or 3 minutes to stabilize their breathing (some subjects hyperventilate initially in respiratory testing).

The respiratory gases preferably comprise oxygen, carbon dioxide and one or more inert gases, such as nitrogen and argon.

The measurements of the amounts of respiratory gases may comprise measurements of the molar flows, or the total flow with concentrations or fractions of respiratory gases in breath. The measurements of the amounts of respiratory gases are preferably made at least every 50ms, more preferably at least every 25ms, more preferably at least every 10ms.

The step of varying the parameters of the lung model to fit the predicted expired respiratory gas amounts to the plural measurements may comprise minimizing the differences, using a non-linear optimisation routine. For example a routine may be used which minimises the sum of the squares of the differences for one or more of the respiratory gases at each expiratory measurement time point. Preferably the step of varying the parameters of the lung model to fit the predicted expired respiratory gas amounts to the plural measurements comprises fitting the predicted expired respiratory gas amounts to the expiratory flow profile over each breath of the multiple successive breaths for one or more of the respiratory gases. In one embodiment the step of varying the parameters of the lung model to fit the predicted expired respiratory gas amounts to the plural measurements comprises fitting the predicted expired carbon dioxide and oxygen amounts measured during the period of steady breathing and fitting the inert gas amounts in the period of inert gas wash-in or wash-out.

A variety of wash-in and/or wash-out procedures may be used. For example, the period of steady breathing may be a period of, e.g., ten minutes, breathing air, this being followed by the period of wash-out by having the subject breathe pure oxygen (or air with added oxygen, e.g. 50%) for, e.g. five minutes, resulting in a wash-out of nitrogen. The model may be fitted to the oxygen and carbon dioxide measurements only during the steady breathing period and to the nitrogen measurements only during the wash-out period. Alternatively, there may be a period of breathing air mixed with a trace gas, such as methane or acetylene, which is also measured, e.g. for five minutes, resulting in a wash-in of the trace gas, followed by a period, e.g. of five minutes, of breathing air, resulting in a wash-out of the trace gas. In this case the model may be fitted to the oxygen, carbon dioxide and one or more trace gas measurements during these periods. Thus the period of steady breathing (i.e. stable oxygen and carbon dioxide) may be simultaneous with the period of inert gas wash-in and/or wash-out. In another approach, the supply of trace gas may be repeatedly added and withdrawn from the inspiratory gases such that wash-in and wash-out periods alternate.

The parameterized lung model models the lung as a plurality of alveolar compartments. These are connected by a respective plurality of personal deadspaces to a common deadspace leading to the mouth. The volumes of the personal deadspaces are distributed with alveolar compartment volume according to a personal deadspace distribution.

In the parameterized model the compartments are defined by one or more of: the volume of the common deadspace; the fractional volume of each alveolar compartment, being the fraction of the total alveolar volume of each compartment at the functional residual capacity of the model; the fractional expansion or compliance of each alveolar compartment, being approximately the fraction of the flow measured at the mouth received by each alveolar compartment; the fractional volume of the personal deadspace for each alveolar compartment, being the fraction of the total personal deadspace; the vascular conductance of each alveolar compartment, being the fraction of the total perfusion received by each compartment.

The model parameters may define: a bivariate distribution for the variation of fractional lung compliance with fractional volume and the variation of vascular conductance with fractional volume, the bivariate distribution being defined by the variance of the fractional lung compliance with fractional volume distribution, the variance of vascular conductance with fractional volume distribution, and their correlation. The bivariate distribution is preferably a lognormal distribution. Preferably in one embodiment the fractional compliance is used as the intrinsic property of the lung, and it is used to approximate the fractional distribution of ventilation.

In one embodiment the model parameters define a normal, or lognormal, distribution for the variation of the fractional personal deadspace volume with the fractional compartment volume.

While lognormal distributions are mentioned above and used in the illustrated embodiments below, other forms of distribution such as normal distributions, or functionally-defined distributions may be used to define the variation of fractional lung compliance with fractional volume, the variation of vascular conductance with fractional volume and the variation of the fractional personal deadspace volume with the fractional compartment volume.

To run the model preferably measurements of the inspired amounts and measured flow of respiratory gases are input to the parametrized model.

Preferably the molecular flow sensor of the apparatus is adapted to measure the amounts of oxygen and carbon dioxide, and preferably also water vapour, in an airway at the mouth of a respiring subject. The molecular flow sensor is preferably adapted to measure the molar flows, or the total flow with concentrations or fractions of respiratory gases in breath of a respiring subject. The molecular flow sensor may also be adapted to measure the amounts of other gases, which may be used as tracer gases, such as methane or acetylene.

In a preferred embodiment the molecular flow sensor is adapted to measure the amounts of respiratory gases at least every 50ms, more preferably at least every 25ms, more preferably at least every 10ms.

The invention also extends to computer program comprising program code means, for example encoded on a tangible storage medium, for controlling a computer to execute the method of the invention.

The invention allows the use of a quantified measure of lung inhomogeneity, preferably determined by the method of the invention, as a biomarker of lung disease. The quantified measure may comprise at least one of: a measure of the variation in lung compliance across the lung, a measure of the variation in deadspace fraction across the lung, a measure of the relative inefficiency of oxygen or carbon dioxide exchange between an inhomogeneous and homogeneous lung, and the difference in systemic arterial or mixed venous oxygen or carbon dioxide concentration between a homogenous and inhomogeneous lung.

The measure of the variation in lung compliance with volume may be the standard deviation or variance in the log of the distribution of lung compliance with alveolar volume and the measure of the variation in deadspace with volume may be the standard deviation or variance of the distribution of deadspace with alveolar volume.

Thus with the present invention inert gas wash-in or wash-out data are used to fit a mathematical model of the lung, producing parameter values that constitute quantitative biomarkers for lung function. In particular, while healthy lungs are relatively homogeneous structures, diseased lungs progressively lose homogeneity. Thus measures of inhomogeneity using the technique of the invention provide biomarkers for chronic airways disease.

The lung model is preferably assembled from a set of individual lung volumes which have associated with them: i) a set of fractional compliance values, which are preferably distributed log-normally; ii) a set of fractional vascular conductance values, which are preferably distributed log-normally; iii) a set of fractional deadspace values, which are preferably distributed normally. The compliance values and vascular conductance values are correlated with each other but the deadspace distribution is preferably not correlated with the compliance and vascular conductive distributions.

The outputs of interest from the invention, which constitute the biomarkers, are: the parameter values of the model together with values derived from them, preferably including: (i) the percent efficiency for gas exchange for carbon dioxide and oxygen compared with a homogenous lung; and (ii) the difference in systemic mixed venous or arterial partial pressures or contents for carbon dioxide and oxygen for the inhomogeneous lung compared with a homogenous lung. The calculation of (i) and (ii) is described below.

> i) Assume the homogeneous lung has a single compartment with the total alveolar volume (at functional residual capacity, FRC) and total deadspace volume. Then compare the gas exchange of the homogeneous lung (under the assumption that the alveolar volume tracks that of the inhomogeneous lung and the venous concentrations remain the same).
> ii) Find the systemic venous concentrations for the homogeneous lung that make the gas exchange for the homogeneous lung match that of the inhomogeneous lung and then compare the differences in venous/arterial concentrations or partial pressures.

In one embodiment, the invention preferably outputs the total volume of personal deadspace, the total alveolar volume, the standard deviation of the personal deadspace distribution, the standard deviation of the log normal compliance:volume distribution and the standard deviation of the log normal vascular conductance:volume distribution.

The present invention will be further described by way of example with reference to the accompanying drawings in which:-

> Figure 1 is an outline of the process of one embodiment of the invention;
> Figure 2 schematically illustrates the apparatus of one embodiment of the invention in use;
> Figure 3 schematically illustrates the basis of the lung model used in one embodiment of the invention;
> Figure 4 shows example measured wash-out data from a wash-out procedure in accordance with an embodiment of the invention;
> Figures 5A to F illustrate distributions for ventilation:perfusion, compliance:volume and vascular conductance:volume ratios for healthy (panels A,C,E) and COPD (panels B,D,F) subjects obtained by an embodiment of the invention;
> Figures 6A and B illustrate distributions for deadspace: volume ratios for healthy and COPD human subjects obtained by an embodiment of the invention;
> Figure 7 illustrates a bivariate log-normal distribution; and
> Figure 8 illustrates a small region of a deadspace compartment in one embodiment of the invention.

This embodiment of the invention is based on the use of a pneumotachograph utilising a laser absorption spectrometer which can provide molecular flow sensing within the airway with an accuracy of better than 0.2% for flow and volume measurement and better than 0.5% for gas analysis, sampling every 10ms. The high precision and high time resolution allow the molar flow of gas species in the airway to be monitored at the subject's mouth and these data are used to fit a mathematical model of the function of an inhomogeneous lung, which in turn provides accurate and new measures of lung inhomogeneity.

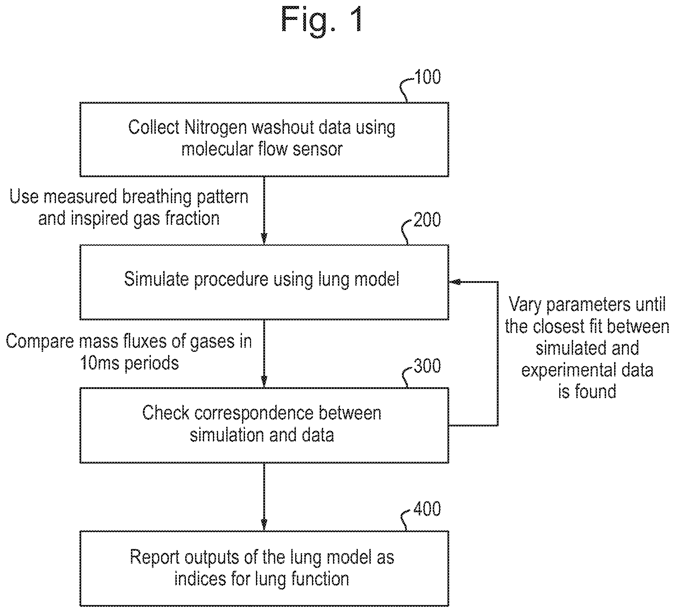

The process of one embodiment of the invention is outlined in Figure 1. In step 100 inert gas wash-out data are collected using a highly accurate, high temporal resolution molecular flow sensor in the airway of a patient. The measured carbon dioxide, oxygen and inert gas flows, together with the subject's arterial oxygen saturation (measured using a pulse oximeter) are transferred to a data processor (such as a general purpose computer) running a simulation 200 of the function of an inhomogeneous lung. The data processor conducts an, e.g. non-linear, optimisation process 300 which finds the sum of the squares of the differences between the gas flows predicted by the simulation 200 and measured experimental values from the procedure 100 of the carbon dioxide and oxygen at each (10ms) time point during an initial phase of air breathing, and the sum of the squares of the differences between the inert gas flows predicted by the simulation 200 and measured during a washout phase following the air breathing phase, and varies the parameters of the mathematical model used in the simulation 200 to minimise sum of the squares of the differences. The data processor reports, in process 400, the outputs and parameters of the mathematical model as indices of lung function.

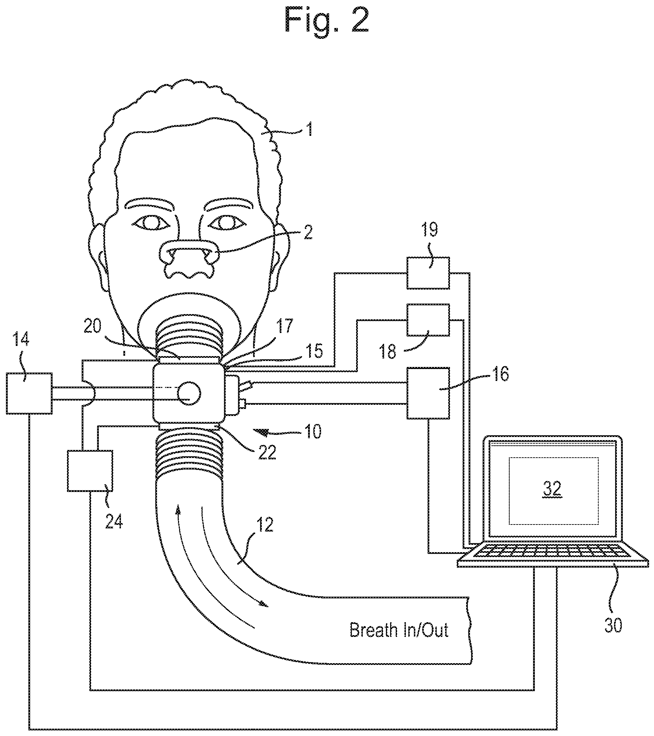

Figure 2 schematically illustrates the apparatus for the procedure 100. This embodiment utilises a molecular flow sensor of the type disclosed in Ciaffoni, L., O'Neill, D. P., Couper, J. H., Ritchie, G. A., Hancock, G., & Robbins, P. A. (2016), "In-airway molecular flow sensing: A new technology for continuous, non-invasive monitoring of oxygen consumption in critical care", Science Advances, 2(8), e1600560. The main functional components of the molecular flow sensor are in a measurement head 10 positioned in an airway 12 of a subject 1. The measurement head 10 is connected to a controller 14 for controlling a laser absorption spectrometer (which is a cavity-enhanced absorption spectrometer) which is used in this embodiment for oxygen measurement, and a controller 16 is provided for controlling a light source and a light detector in a separate optical path used for carbon dioxide and water concentration measurements. On either side of the measurement head 10 are pressure sampling assemblies 20 and 22 connected to a differential pressure sensor 24. The measurement head 10 also includes a temperature sensor 15 for measuring the temperature of the gas flowing through the airway, and a barometric pressure sensor 17 for measuring the static pressure inside the measurement space, connected to respective controllers 18 and 19. The individual controllers 14, 16, 18, 19 and 24 are connected to a system controller 30 which controls the individual controllers, receives their outputs and calculates their results. The results may be displayed on display 32 and in this embodiment are output to a data processor. In particular the outputs are fluxes in litres (STPD) of oxygen, carbon dioxide, water vapour and balance (inert) gas every 10ms.

Alternative embodiments may include sensors to measure other inert gases such as methane or acetylene which may be used as trace gases and in such cases the respiratory measurement procedure 100 of Figure 1 alternatively uses such a trace gas as the inert gas in a wash-in followed by wash-out procedure, or alternating periods of wash-in and washout. In these cases the measurements of the inert gas may be simultaneous with the measurements of carbon dioxide and oxygen.

In practice a subject may need to be allowed to breathe steadily for 2 or 3 minutes to stabilize their breathing (some subjects hyperventilate initially in respiratory testing). Thus measurements during that period are not used. After the subject has settled-down a period of steady breathing may be used to set the model's initial conditions without varying the parameters of the lung model. Then the period of steady breathing followed by the period of inert gas wash-out may follow, or the periods of wash-in and wash-out of inert gases, with the gas flows being accurately measured for supply of the measurements to the data processor.

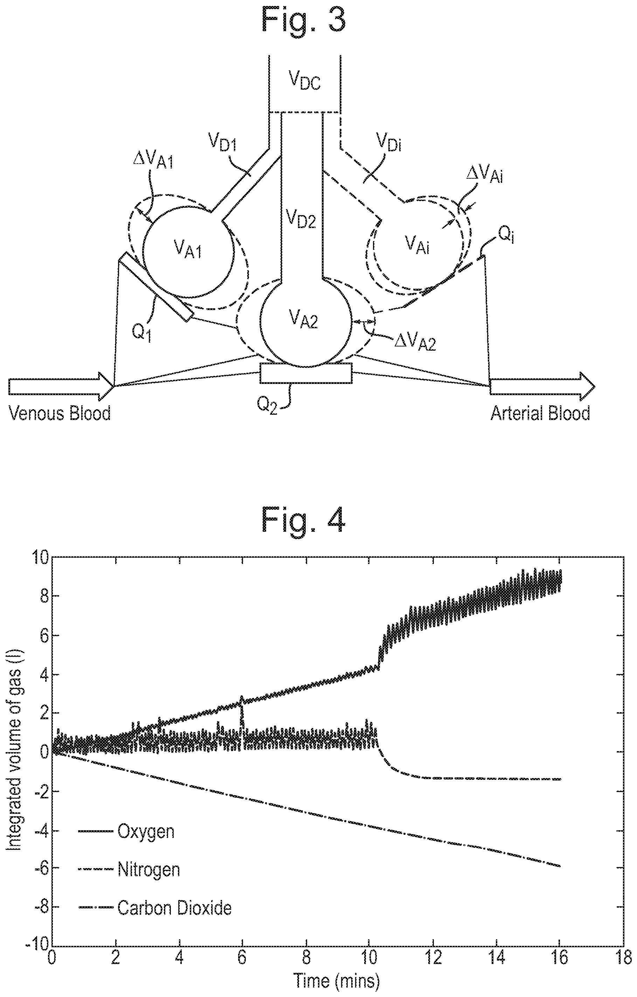

Figure 4 illustrates example wash-out data output from the apparatus for a 16 minute period in which for the first 10 minutes the subject breathes constant-inspired oxygen and carbon dioxide (here, atmospheric air) and a subsequent 6 minute period of wash-out of an inert gas (mainly nitrogen) by breathing pure oxygen.

Figure 3 illustrates the components of the inhomogeneous lung model used in this embodiment. To model heterogeneity within the lung, a lung of total alveolar volume $V_A$ is constructed from a set of N lung compartments of equal volume (at FRC) (only three lung compartments are illustrated but in a typical model there would be many - e.g. N=125), but which differ in their fractional shares of: total lung compliance ($C_L$) (compliance is the change in volume divided by the change in pressure), total pulmonary vascular conductance ($C_d$) (conductance is the blood flow per unit of driving pressure) and total deadspace ($V_D$). By using fractional quantities (i.e. the total is normalized to one) it is not necessary to look at absolute values for each compartment. The lung compartments are connected to a common deadspace $V_{DC}$ and in turn to the molecular flow sensor, which has its own (known) deadspace.

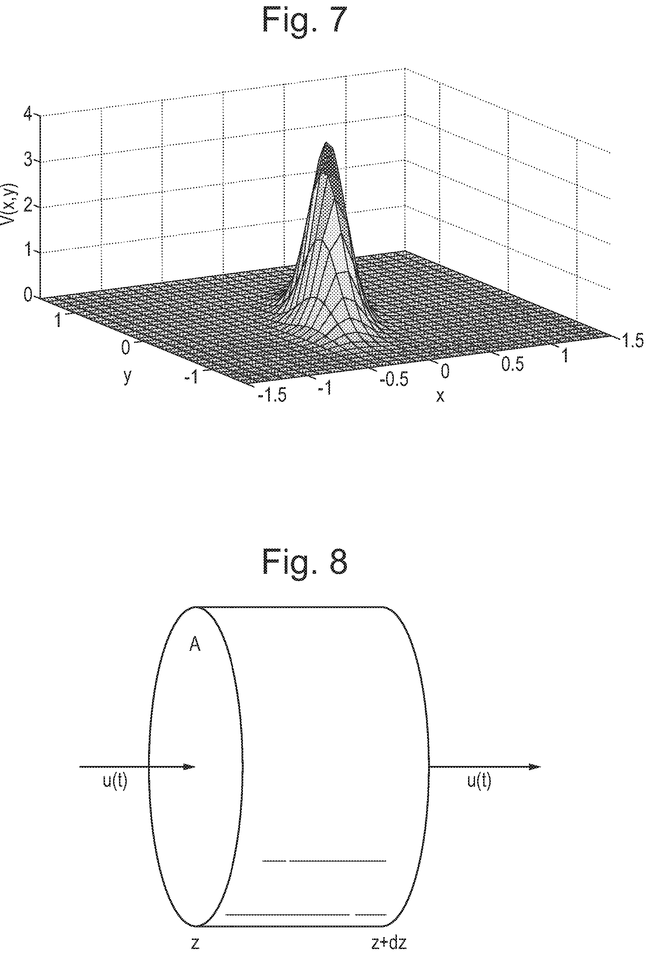

To minimise the parameter count it is assumed based on previous studies (Wilson, JAP 72: 2298-2304, 1992; Beck JAP 90: 2151-2156, 2001; Beck JAP 113: 872-877, 2012), that both $C_L$ and Cd have a continuous log-normal distribution at FRC. This results in a bivariate log-normal distribution for these parameters as illustrated in Figure 7, where the correlation between the two distributions is defined as $\rho$. The standard deviations of $C_L$ and Cd are $\sigma_{CL/V_A}$ and $\sigma_{Cd/V_A}$, respectively. The variation of deadspace between compartments is described by a normal distribution with standard deviation $\sigma_{V_D}$. We explain below how this continuous distribution is discretised into discrete lung compartments. The final core parameter of the lung required is the volume multiplier (Vtiss) for $CO_2$, which accounts for the additional effective lung volume that arises from the solubility of $CO_2$ within the lung tissue (Cander JAP 14: 541-551, 1959; Sackner JAP 19: 374-380, 1964). These parameters and their definitions are listed in Table 1.

**Table 1**

| Parameter | Definition |
|---|---|
| $V_A = N V_{Ai}$ | Total alveolar volume when the lungs are at FRC, where N is the number of lung compartments. |
| $V_{\text{tiss}} = \dfrac{V_{ACo_2} - V_A}{V_A}$ | Volume multiplier for carbon dioxide to incorporate the effective lung tissue volume |

(continued)

| Parameter | Definition |
|---|---|
| $$Q_T = \sum_{i=1}^{N} Q_i$$ | Cardiac output ($Q_i$ is the blood flow associated with compartment $i$) |
| $$\sigma_{C_L/V_A} = \mathrm{SD}\left(\ln\left(\frac{N\Delta V_{Ai}}{\sum \Delta V_{Ai}}\right)\right)$$ | Standard deviation of fractional compliance ($C_L$) distribution; where: $$C_L = \sum C_{Li} = 1 \text{ and } C_{Li} = \frac{\Delta V_{Ai}}{\sum \Delta V_{Ai}}$$ |
| $$\sigma_{C_d/V_A} = \mathrm{SD}\left(\ln\left(\frac{NQ_i}{\sum Q_i}\right)\right)$$ | Standard deviation of fractional vascular conductance $C_d$ distribution; where: $$C_d = \sum C_{di} = 1 \text{ and } C_{di} = \frac{Q_i}{\sum Q_i}$$ |
| $$\rho = \mathrm{corr}\left(\ln\left(\frac{N\Delta V_{Ai}}{\sum \Delta V_{Ai}}\right), \ln\left(\frac{NQ_i}{\sum Q_i}\right)\right)$$ | Correlation of vascular conductance and compliance |
| $V_{DC}$ | Apparatus deadspace volume |
| $$V_D = \sum_{i=1}^{N} V_{Di}$$ | Total personal deadspace volume |
| $$\sigma_{VD} = \frac{N}{V_D}\mathrm{SD}(V_{Di})$$ | Standard deviation of deadspace distribution |

To run the model, an alternating inspiratory and expiratory respiratory flow pattern and a pulmonary blood flow must be supplied. It also needs to be supplied with an inspiratory gas composition and a method for calculating pulmonary arterial blood gas composition (which will be explained below). The outputs of the model are the compositions of the expired gas during exhalation and the composition of pulmonary venous blood throughout the respiratory cycle. A fuller description of the model and the estimation of its parameters is given below.

**Detailed structure of the model and description of parameter estimation procedure**

The fractional compliance ($C_{Li}$) and fractional pulmonary vascular conductance ($C_{di}$) of an alveolar compartment are determined by discretising a continuous, bivariate lognormal distribution.

**Bivariate lognormal distribution**

For ease of notation $x$ and $y$ are defined as:

$$x = \ln\left(\frac{C_L(x)}{V(x,y)}\right) \qquad \text{(E1)}$$

$$y = \ln\left(\frac{C_d(y)}{V(x,y)}\right) \ ,$$

where $C_L(x)$ is the fractional compliance, $C_d(y)$ is the fractional pulmonary vascular conductance, and $V(x,y)$ is the fractional alveolar volume at FRC.

The governing distribution of the model $V(x,y)$ is defined with the statement that $V(x,y)dxdy$ is the fraction of the alveolar volume with $x = \log\left(\frac{C_L}{V}\right)$ and $y = \log\left(\frac{C_d}{V}\right)$ values that lie in small intervals of length $dx$ and $dy$ around the values of $x$ and $y$.

It is assumed that V is a bivariate lognormal distribution of $\frac{C_L}{V}$ and $\frac{C_d}{V}$, thus:

$$V(x,y) = \frac{1}{2\pi\sigma_{C_L/V_A}\sigma_{C_d/V_A}\sqrt{1-\rho^2}} \exp\left(\frac{-1}{2(1-\rho^2)}\left(\frac{(x-\bar{x})^2}{\sigma_{C_L/V_A}^2} - \frac{2\rho(x-\bar{x})(y-\bar{y})}{\sigma_{C_L/V_A}\sigma_{C_d/V_A}} + \frac{(y-\bar{y})^2}{\sigma_{C_d/V_A}^2}\right)\right), \qquad (E2)$$

where $\sigma_{C_L/V_A}^2$ and $\sigma_{C_d/V_A}^2$ are the variances of the compliance-volume and vascular conductance-volume distributions respectively, $\bar{x}$ and $\bar{y}$ are their means, and $\rho$ the coefficient of correlation between $x$ and $y$.

The compliance-volume ($V(x)$) and vascular conductance-volume ($V(y)$) distributions are the projections of V($x$, $y$) onto the $x$ and $y$ axes respectively. Thus,

$$V(x) = \int_{-\infty}^{+\infty} V(x,y)dy, \qquad (E3)$$

and

$$V(y) = \int_{-\infty}^{+\infty} V(x,y)dx, \qquad (E4)$$

Substituting (E2) into these expressions we have

$$V(x) = \frac{1}{\sqrt{2\pi}\sigma_{C_L/V_A}} \exp\left(\frac{(x-\bar{x})^2}{\sigma_{C_L/V_A}^2}\right), \qquad (E5)$$

and

$$V(y) = \frac{1}{\sqrt{2\pi}\sigma_{C_d/V_A}} \exp\left(\frac{(y-\bar{y})^2}{\sigma_{C_d/V_A}^2}\right). \qquad (E6)$$

Thus V($x$) and V($y$) are univariate lognormal distributions.
As will now be shown, this distribution is described by only three independent parameters. From (E1),

$$C_L(x) = V(x)e^x, \qquad (E7)$$

substituting (E5) into this expression and rearranging:

$$C_L(x) = \frac{1}{\sqrt{2\pi}\sigma_{C_L/V_A}} \exp\left(\frac{-(x-(\bar{x}+\sigma_{C_L/V_A}^2))^2}{2\sigma_{C_L/V_A}^2}\right) \exp\left(\frac{\left((\sigma_{C_L/V_A}^2 - 2\bar{x})\right)}{2}\right). \qquad (E8)$$

Since it is required that

$$\int_{-\infty}^{+\infty} C_L(x)dx = 1, \qquad (E9)$$

then

$$\exp\left(\frac{\sigma_{C_L/V_A}^2 + 2\bar{x}}{2}\right) = 1, \qquad (E10)$$

and so

$$\bar{x} = -\frac{\sigma^2_{C_{L/V_A}}}{2}. \qquad (E11)$$

Since from (E1),

$$C_d(y) = V(y)e^y, \qquad (E12)$$

an analogous procedure gives:

$$\bar{y} = -\frac{\sigma^2_{C_{d/V_A}}}{2}. \qquad (E13)$$

In contrast to previous work the means of the distribution, $\bar{x}$ and $\bar{y}$, are not independent parameters, and the distribution is governed by three parameters: $\sigma_{C_{L/V_A}} \sigma_{C_{d/V_A}}$ and $\rho$. This is because fractional quantities are being used, and the distribution is assumed as independent of the ventilation and the cardiac output.

The distribution (E2) must be discretized to provide the compliances and conductances for each of the compartments of the model with $N_x$ $N_y$ unique pairs of compliance-volume and vascular conductance-volume ratios. Each of these pairs is indexed with the subscripts $ij$, where $i = 1,2,...N_x$ and $j = 1,2,...N_y$. Various methods may be used to discretize the distribution, but two methods are described below.

**A straightforward discretisation**

First $x$ and $y$ are discretised into $N_x$ +1 and $N_y$+1 equally spaced points. Since these points are equally spaced finite upper and lower bounds must be chosen, and thus $x_0$ is set such that

$$\int_{-\infty}^{x_0} V(x)dx = 0.0001, \qquad (E14)$$

and $x_{N_x}$ such that

$$\int_{x_{N_x}}^{\infty} V(x)dx = 0.0001. \qquad (E15)$$

The values $y_0$ and $y_{N_y}$ are defined by analogous expressions involving the relevant univariate distribution V($y$).
The fractional volume of compartment ($ij$) when the lung is at FRC can then be defined as,

$$V_{i,j} = \int_{y_{j-1}}^{y_j} \int_{x_{i-1}}^{x_i} V(x,y)dxdy, \qquad (E16)$$

Using the definitions of $x$ and $y$ given by (E1), the compliance ($C_{L_{i,j}}$) is

$$C_{L_{i,j}} = \int_{y_{j-1}}^{y_j} \int_{x_{i-1}}^{x_i} \exp(x)V(x,y)dxdy, \qquad (E17)$$

and the vascular conductance $C_{d_{i,j}}$,

$$C_{d_{i,j}} = \int_{y_{j-1}}^{y_j} \int_{x_{i-1}}^{x_i} \exp(y)V(x,y)dxdy. \qquad (E18)$$

This method of discretising the continuous distribution works particularly well if the major and minor axes of the elliptical probability density function lie along the x and y axes (i.e. if $\rho$=0). However if $\rho \neq 0$ the probability density function is an ellipse whose major and minor axes do not lie along the x and y axes. In this case the approach described above, in which the distribution is discretized into equal-sized rectangles along the x and y axes, will be quite inefficient since there will be a number of compartments with almost zero volume, and one or two containing the majority of the volume.

Thus in the next section advantage is taken of a geometrical property of the normal distribution that allows a change of variables so that the distribution can be discretised more efficiently.

### Discretisation by change of variables

To discretise the governing continuous distribution more efficiently, advantage is taken of a useful property of the bivariate normal distribution. This property is that any bivariate normal distribution can be represented as a transformation of the standard bivariate normal distribution (zero means, zero covariance, and unit variances). In this section the outline given in "The multivariate Gaussian distribution" by C. B. Do is followed, and we explain how this change of variables may be made by formulating the bivariate normal distribution in vector notation. The integrals that must be solved to define the compartments in these new variables are then set-up.

The bivariate distribution, (E2), can be written in the following notation:

$$V(\mathbf{x}) = \frac{1}{2\pi|\Sigma|^{\frac{1}{2}}} \exp\left(-\frac{1}{2}(\mathbf{x}\text{-}\mu)^T \Sigma^{-1}(\mathbf{x}\text{-}\mu)\right), \qquad (E19)$$

where

$$\mathbf{x} = \begin{pmatrix} x \\ y \end{pmatrix}, \qquad (E20)$$

$$\mu = \begin{pmatrix} \bar{x} \\ \bar{y} \end{pmatrix}, \qquad (E21)$$

$$\Sigma = \begin{pmatrix} \sigma^2_{C_L:V_A} & \rho\sigma_{C_L:V_A}\sigma_{C_d:V_A} \\ \rho\sigma_{C_L:V_A}\sigma_{C_d:V_A} & \sigma^2_{C_d:V_A} \end{pmatrix}. \qquad (E22)$$

The contour lines of constant probability are ellipses centred on the mean, with major and minor axes defined by the eigenvectors of $\Sigma$.

To perform a change of variables we first diagonalise $\Sigma$,

$$\Sigma = \mathbf{U}\Lambda\mathbf{U}^\mathbf{T}, \qquad (E23)$$

where $\Lambda$ is a diagonal matrix with, as $0 \leq \rho \leq 1$, positive entries on the diagonal and $\mathbf{U}$ is a rotation matrix, and so

$$\mathbf{U}\mathbf{U}^\mathbf{T} = I, \qquad (E24)$$

where $I$ is the identity matrix. We can re-write (E23) as

$$\Sigma = (\mathbf{U}\Lambda^{\frac{1}{2}})(\mathbf{U}\Lambda^{\frac{1}{2}})^\mathbf{T}, \qquad (E25)$$

where

$$\Lambda^{\frac{1}{2}} = \begin{pmatrix} \sqrt{\Lambda_{11}} & 0 \\ 0 & \sqrt{\Lambda_{22}} \end{pmatrix}. \qquad (E26)$$

If we substitute (E25) into (E19) and use the fact that the determinant of a matrix's transpose is equal to the determinant of the matrix, we have

$$V(\mathbf{x}) = \frac{1}{2\pi \left| (\mathbf{U}\Lambda^{\frac{1}{2}}) \right|} \exp\left( -\frac{1}{2}(\mathbf{x}\text{-}\boldsymbol{\mu})^T (\mathbf{U}\Lambda^{\frac{1}{2}})^{-T}(\mathbf{U}\Lambda^{\frac{1}{2}})^{-1}(\mathbf{x}\text{-}\boldsymbol{\mu}) \right). \quad \text{(E27)}$$

To simplify this expression we define a new set of axes $\mathbf{s}$,

$$\mathbf{s} = (\mathbf{U}\Lambda^{\frac{1}{2}})^{-1}(\mathbf{x}\text{-}\boldsymbol{\mu}), \qquad \text{(E28)}$$

where

$$\mathbf{s} = \begin{pmatrix} s \\ t \end{pmatrix}, \qquad \text{(E29)}$$

and thus multiplying (E28) by $\mathbf{U}\Lambda^{\frac{1}{2}}$ we have

$$\mathbf{x} = \boldsymbol{\mu} + \mathbf{U}\Lambda^{\frac{1}{2}}\mathbf{s}, \qquad \text{(E30)}$$

The Jacobian of this transformation is $\mathbf{J}$, where

$$J = \begin{pmatrix} \frac{\partial x}{\partial s} & \frac{\partial x}{\partial t} \\ \frac{\partial y}{\partial s} & \frac{\partial y}{\partial t} \end{pmatrix}. \qquad \text{(E31)}$$

Calculating this explicitly from (E30) we have that,

$$J = \mathbf{U}\Lambda^{\frac{1}{2}}. \qquad \text{(E32)}$$

To change the variables of a probability density function (PDF), the PDF in the old variables is multiplied by the determinant of the Jacobian for the transformation between the two sets of variables. Thus in our case,

$$V(\mathbf{s}) = V(\mathbf{x})|\mathbf{J}|. \qquad \text{(E33)}$$

Using this and substituting for $\mathbf{s}$ in (E27), we obtain

$$V(\mathbf{s}) = \frac{1}{2\pi} \exp(-\frac{1}{2}\mathbf{s}^{\mathbf{T}}\mathbf{s}). \qquad \text{(E34)}$$

This is the standard bivariate normal distribution (zero means, zero covariance, and unit variances) defined on s. Thus any bivariate normal distribution defined on $\mathbf{x}$ can be represented as the standard bivariate normal distribution on $\mathbf{s}$, using a suitable change of variables. The advantage of using the standard bivariate normal distribution is that the major axes of the ellipses that form the probability density function are along the axes on which we define the distribution.

We can then define a new rectangular grid on $s$ and $t$, with $N_s$ intervals in the s direction and $N_t$ intervals in the t direction, which samples the distribution effectively, and replace the integrals discussed above to find the compliances, volumes, and vascular conductances associated with these ($N_s N_t$) rectangles (compartments).

In the new variable $s$ and $t$ we can replace (E16) with

$$V_{ij} = \int_{t_{j-1}}^{t_j} \int_{s_{i-1}}^{s_i} V(s,t)\,ds\,dt, \qquad \text{(E35)}$$

where $i = 1 \ldots N_s$, $j = 1 \ldots N_t$, and

$$V(s,t) = \frac{1}{2\pi} \exp\left(\frac{-s^2 - t^2}{2}\right). \qquad \text{(E36)}$$

We can factorise (E35) into the product of two univariate integrals. If we do this we have

$$V_{ij} = \int_{t_{j-1}}^{t_j} \frac{1}{\sqrt{2\pi}} \exp\left(\frac{-t^2}{2}\right) \mathrm{d}t \int_{s_{i-1}}^{s_i} \frac{1}{\sqrt{2\pi}} \exp\left(\frac{-s^2}{2}\right) \mathrm{d}s. \qquad \text{(E37)}$$

In the new variables we can then define the limits on the integrals such that each compartment represents an equal fraction of the alveolar volume at FRC (i.e. $V_{ij} = \frac{1}{N_s N_t}$ ), rather than the compartments defined in the first discretization scheme above which represented equally sized rectangles on $x$ and $y$. To do this we define $s_i$ such that

$$\int_{s_{i-1}}^{s_i} \frac{1}{\sqrt{2\pi}} \exp\left(\frac{-s^2}{2}\right) \mathrm{d}s = \frac{1}{N_s}. \qquad \text{(E38)}$$

In the new variables the limits are no longer equally spaced, and so $s_0 = -\infty$ and $s_{N_s} = \infty$. Similarly we define $t_j$ such that

$$\int_{t_{j-1}}^{t_j} \frac{1}{\sqrt{2\pi}} \exp\left(\frac{-t^2}{2}\right) \mathrm{d}t = \frac{1}{N_t}, \qquad \text{(E39)}$$

where $t_0 = -\infty$ and $t_{N_t} = \infty$.
Changing the variables of (E7) using (E30) and (E32) we have,

$$C_L(s,t) = \exp(\bar{x} + J_{11}s + J_{12}t)V(s,t). \qquad \text{(E40)}$$

Thus integrating between the limits on $s$ and $t$ we can discretise this expression to define the fractional compliance of compartment $ij$ ($C_{Lij}$) as

$$C_{Lij} = \int_{t_{j-1}}^{t_j} \int_{s_{i-1}}^{s_i} \exp(\bar{x} + J_{11}s + J_{12}t)V(s,t)\mathrm{d}s\mathrm{d}t. \qquad \text{(E41)}$$

Similarly changing the variables of (E12) using (E30) and (E32), and integrating the fractional vascular conductance of compartment $ij$($C_{dij}$) is

$$C_{dij} = \int_{t_{j-1}}^{t_j} \int_{s_{i-1}}^{s_i} \exp(\bar{y} + J_{21}s + J_{22}t)V(s,t)\mathrm{d}s\mathrm{d}t. \qquad \text{(E42)}$$

**Discretisation of the deadspace**

The total volume of personal deadspace associated with each of the $N_s N_t$ unique pairs of compliance-volume and vascular conductance-volume ratios is $V_{ij}V_D$. However, alveolar compartments with the same compliance-volume and vascular conductance-volume ratios may be located in different regions of the lung. Thus they will be associated with different volumes of deadspace. In this embodiment it is assumed that rather than forming a single compartment, this deadspace is also distributed, according to a personal deadspace distribution.

This distribution is discretised into $N_d$ compartments for each pair of compliance-volume and vascular conductance-volume ratios; thus the total number of both alveolar and deadspace compartments, $N$, is $N_s N_t N_d$. The deadspace distribution is assumed to be a normal distribution and identical for each pair of compliance-volume and vascular conductance-volume ratios.

Defining:

$$z = \frac{v_{ij}}{V_{ij}}, \qquad \text{(E43)}$$

where $v_{ij}$ is the fractional deadspace volume of an alveolar compartment ( $\dfrac{V_{Di}}{V_D}$ ), and thus

$$v_{ij} = z V_{ij},\qquad\qquad\text{(E44)}$$

Assuming the fractional volume is normally distributed,

$$V_{ij}(z) = \frac{V_{ij}}{2\pi\sigma_{V_D}}\exp\left(\frac{-(z-\overline{z_{ij}})^2}{2\sigma_{V_D}^2}\right),\qquad\qquad\text{(E45)}$$

Substituting (E43) into this expression :

$$v_{ij}(z) = \frac{V_{ij}z}{\sqrt{2\pi}\sigma_{V_D}}\exp\left(\frac{-(z-\overline{z_{ij}})^2}{2\sigma_{V_D}^2}\right).\qquad\qquad\text{(E46)}$$

The total fractional deadspace volume associated with each unique pair of $C_{Lij}/V_{ij}$ and $C_{dij}/V_{ij}$ ratios is $V_{ij}$. Thus :

$$\int_{-\infty}^{\infty} v_{ij}(z)dz = V_{ij}.\qquad\qquad\text{(E47)}$$

Letting $= z - \overline{z_{iJ}}$ :

$$\int_{-\infty}^{\infty}\frac{V_{ij}(Z+\overline{z_{ij}})}{\sqrt{2\pi}\sigma_{V_D}}\exp\left(\frac{-Z^2}{2\sigma_{V_D}^2}\right)dZ = V_{ij},\qquad\qquad\text{(E48)}$$

hence

$$V_{ij} = \overline{z_{iJ}}V_{ij} + \int_{-\infty}^{\infty}\frac{V_{ij}Z}{\sqrt{2\pi}\sigma_{V_D}}\exp\left(\frac{-Z^2}{2\sigma_{V_D}^2}\right)dZ,\qquad\qquad\text{(E49)}$$

and since the integrand is an odd function :

$$\bar{z}_{ij} = 1.\qquad\qquad\text{(E50)}$$

Thus the normal deadspace distribution introduces one additional parameter, $\sigma_{V_D}$.
The continuous deadspace distribution, (E46), is now discretized into discrete compartments. As each of the $N_sN_t$ compartments (denoted with indices $ij$) is split into $N_d$ compartments, a third index $k$ is introduced. Again assuming that each alveolar compartment has equal volume, the fractional volume of compartment $ijk$, $V_{ijk}$, is

$$V_{ijk} = \int_{z_{k-i}}^{z_k} V_{ij}(z)dz = \frac{V_{ij}}{N_d},\qquad\qquad\text{(E51)}$$

with $z_0 = -\infty$ and $z_{N_d} = \infty$.
The fractional deadspace volume ($v_{ijk}$) associated with compartment $ijk$ can then be defined as

$$v_{ijk} = \int_{z_{k-i}}^{z_k} v_{ij}(z)dz.\qquad\qquad\text{(E52)}$$

The vascular conductance-volume and compliance-volume ratios of the $N_sN_t$ compartments (denoted with indices $ij$) do not change when each of them is split into $N_d$ compartments (as these are set by the governing alveolar distribution), and thus we have

$$C_{dijk} = C_{dij}\frac{v_{ijk}}{v_{ij}} \qquad\qquad (E53)$$

Similarly the fractional compliance is

$$C_{Lijk} = C_{Lij}\frac{v_{ijk}}{v_{ij}} \qquad\qquad (E54)$$

The normal deadspace distribution (E45) is defined from $-\infty<z<\infty$. From (E43) we see that $z<0$ gives negative values of $\frac{v}{V}$. Thus if the value of $\sigma_{V_D}$ is sufficiently large (the larger the value of $\sigma_{V_D}$ the greater the density of the distribution away from its mean of one), then (E52) may give a deadspace compartment with a negative volume, which is clearly not physically realistic. Thus a maximum value for $\sigma_{V_D}$ is set that prevents (E52) giving a compartment with a negative volume.

**Note on indexing of compartments**

This section began referring to compartments with two indices, *ij,* due to the discretisation of the bivariate lognormal distribution. A third index, *k*, was introduced as each one of these *ij* compartments was split into $N_d$ compartments due to the discretisation of the deadspace distribution. Thus each compartment had three indices, *ijk*, and the total number of compartments is $N_s{\times}N_t{\times}N_d$.

This indexing was convenient for explaining the discretisation of the distributions. However, for simplicity of notation, in the rest of the document each compartment is uniquely identified with a single index î, where

$$\hat{\imath}=i+N_s(j-1)+N_sN_t(k-1), \qquad\qquad (E55)$$

where

$$i= \quad 1,2,\ldots,N_s,$$

$$j= \quad 1,2,\ldots,N_t,$$

$$k= \quad 1,2,\ldots,N_d.$$

In the rest of the document the ^ is dropped and *i* (given by (E55)) will be the sole index used to refer to the compartments.

**Governing equations**

In this section the governing equation of the alveolar compartments for this embodiment will first be described, followed by how the fractional ventilation (the fraction of the flow measured at the mouth entering/leaving an alveolar compartment) is calculated from the compartment's compliance. The governing equations of the body's gas stores and deadspace compartments used in this embodiment are then given.

**Alveolar Compartments**

As indicated above, it is assumed that the alveolar compartments are perfectly mixed, and that the gas exchange between the blood and the alveolar compartment reaches equilibrium. Since $N_2$ and $O_2$ are almost insoluble in lung tissue it is assumed that the volume of these gases dissolved in lung tissue will be negligible. A similar approach can be taken for any other relatively insoluble tracer gases that may be present. By conservation of mass, the rate of change of the volume of a given gas in a lung compartment is equal to the sum of the flux of that gas entering via ventilation from the personal deadspace, and the net flux of that gas due to exchange with the blood across the alveolar membrane. Thus for $N_2$ and $O_2$ during inspiration:

$$\frac{d(F_i^g(t)V_{Ai}(t))}{dt} = \dot{V}_t^{in}u(t)F_{Ii}^g(t) + Q_T C_{di}\left(C_{\bar{v}}^g(t) - C_{ai}^g(t)\right), \qquad (E56)$$

where $F_i^g(t)$ is the gas fraction g (where g may be $O_2$ or $N_2$) in compartment *i* at time *t*, $\dot{V}_t^{in}$ is the fraction of the flow measured at the mouth that enters compartment *i* during inspiration (the calculation of this quantity is discussed below), $F_{Ii}^g(t)$ is the fraction of gas *g* inspired into compartment *i* from the connected personal deadspace compartment, $Q_T$ is the cardiac output, $C_{\bar{v}}^g(t)$ is the mixed venous (pulmonary arterial) gas concentration of gas *g* entering the compartments, $C_{ai}^g(t)$ the blood gas concentration of gas g leaving compartment *i*, $V_{Ai}(t)$ the volume of compartment *i*, and *u(t)* is the flow rate of gas (measured by the in-airway molecular flow sensing device (MFS)). It should be noted that since gas exchange with the blood is considered, $\frac{dV_{Ai}(t)}{dt}$ is not equal to $\dot{V}_t^{in}u(t)$, as is often assumed, but is given by (E35), which is derived below.

However, $CO_2$ is significantly more soluble than $N_2$ or $O_2$, and thus is present in appreciable volumes in lung tissue. It is assumed that the tissue volume is a constant fraction (*b*) of the total alveolar volume at FRC ($V_A$) and distributed among the compartments in proportion to their volume at FRC. It is assumed that the partial pressures of $CO_2$ in the tissue and the alveoli are at equilibrium, and thus the volume of $CO_2$ dissolved in the tissue associated with compartment *i* is

$$V_i V_A b \lambda_t (P_b - P_{H2O}) F_i^{CO_2}(t), \qquad (E57)$$

where $\lambda_t$ is the solubility of $CO_2$ in lung tissue. The volume multiplier for $CO_2$ to reflect the effective lung tissue volume within which $CO_2$ is distributed, $V_{tiss}$, is then defined as

$$V_{tiss} = b\lambda_t(P_b - P_{H2O}). \qquad (E58)$$

An analogous approach may be adopted for any soluble tracer gases that may be present.

Therefore, including this volume, for $CO_2$ on inspiration, by analogy with (E56):

$$\frac{d(F_i^{CO_2}(t)(V_{Ai}(t)+V_i V_{tiss}V_A))}{dt} = \dot{V}_i^{in}u(t)F_{Ii}^{CO_2}(t) + Q_T C_{di}\left(C_{\bar{v}}^{CO_2}(t) - C_{ai}^{CO_2}(t)\right), \qquad (E59)$$

Summing (E56) (for both $N_2$ and $O_2$) and (E59), re-arranging, and remembering that $F^{CO2}+F^{N2}+F^{O2}=1$, gives:

$$\frac{dV_{Ai}(t)}{dt} = \dot{V}_i^{in}u(t) + Q_T C_{di} \sum_g \left(C_{\bar{v}}^g(t) - C_{ai}^g(t)\right) - V_i V_{tiss}V_A \frac{dF_i^{CO_2}(t)}{dt}, \qquad (E60)$$

where *g* denotes one of the three respiratory gases. Thus as noted earlier $\frac{dV_{Ai}(t)}{dt}$ is not equal to $\dot{V}_i^{in}u(t)$.

On expiration the flux due to ventilation is leaving rather than entering the compartment. Thus (E56) becomes:

$$\frac{d(F_i^g(t)V_{Ai}(t))}{dt} = \dot{V}_i^{ex}u(t)F_i^g(t) + Q_T C_{di}(C_{\bar{v}}^g(t) - C_{ai}^g(t)), \qquad (E61)$$

where $\dot{V}_i^{ex}$ is the fraction of the flow measured at the mouth that leaves compartment *i* during expiration (the calculation of this quantity is discussed below), and *g* is $O_2$ or $N_2$. Similarly for $CO_2$ :

$$\frac{d(F_i^{CO_2}(t)(V_{Ai}(t)+V_i V_{tiss}V_A))}{dt} = \dot{V}_i^{ex}u(t)F_i^{CO_2}(t) + Q_T C_{di}(C_{\bar{v}}^{CO_2}(t) - C_{ai}^{CO_2}(t)). \quad (E62)$$

Summing (E61) (for both $N_2$ and $O_2$) and (E62), and re-arranging, gives the governing equation for compartmental volumes on expiration:

$$\frac{dV_{Ai}(t)}{dt} = \dot{V}_i^{ex} u(t) + Q_T C_{di} \sum_g \left( C_{\bar{v}}^g(t) - C_{ai}^g(t) \right) - V_i V_{\text{tiss}} V_A \frac{dF_i^{CO_2}(t)}{dt}. \quad \text{(E63)}$$

**Fractional ventilation**

We assume constant values for $\dot{V}_i$ (the fraction of the flow measured at the mouth entering/leaving an alveolar compartment) during both expiration and inspiration, although the values for expiration and inspiration will be different, and are updated on a breath by breath basis as explained below.

The values for $\dot{V}_i$ are initialized by setting them equal to the values of $C_{Li}$, since the volume change during a single breath due to ventilation is much larger than that due to net gas exchange with the blood. On subsequent breaths, the value of $\dot{V}_i$ during inspiration ( $\dot{V}_i^{in}$ ) is the value that would have led the following integral to hold on the previous breath:

$$\int_0^{t_I} \frac{dV_{Ai}(t)}{dt} dt = \int_0^{t_I} C_{Li} \frac{dV_{Ai}(t)}{dt} dt, \quad \text{(E64)}$$

where $t_I$ is the duration of that inspiration. Substituting in (E60), integrating, and re-arranging we have:

$$\dot{V}_i^{in} = \frac{C_{Li}\left(V_A(t_I) - V_A(0)\right) - \int_0^{t_I} Q_T C_{di} \sum_g \left( C_{\bar{v}}^g(t) - C_{ai}^g(t) \right) + V_i V_{\text{tiss}} V_A \frac{dF_i^{CO_2}(t)}{dt} dt}{\int_0^{t_I} u(t) dt}. \quad \text{(E65)}$$

Analogously on expiration:

$$\dot{V}_i^{ex} = \frac{C_{Li}\left(V_A(t_b) - V_A(t_I)\right) - \int_{t_I}^{t_b} Q_T C_{di} \sum_g \left( C_{\bar{v}}^g(t) - C_{ai}^g(t) \right) + V_i V_{\text{tiss}} V_A \frac{dF_i^{CO_2}(t)}{dt} dt}{\int_{t_I}^{t_b} u(t) dt}. \quad \text{(E66)}$$

The values of these expression are estimated at the end of each breath and the calculated values of $\dot{V}_i^{in}$ and $\dot{V}_i^{ex}$ used on the subsequent inspiration/expiration.

**The body's gas stores**

Above are derived the governing equations for the alveolar compartments, and it can be seen that they depend on the venous gas concentrations $C_{\bar{v}}^g(t)$. During the air breathing phase of the washout procedure 100, these values are relatively stable and can be calculated from the data without the need for a model of recirculation. In the case of a nitrogen washout procedure using a high content of, or pure, oxygen as the inspiratory gas, the concentrations of both $N_2$ and $O_2$ in the pulmonary venous blood change substantially, and so models for recirculation must be employed.

To model the body's inert gas stores, in this embodiment the nitrogen stores, and their exchange with the blood, a known model (and parameters) such as that of e.g. Baker and Farmery, Comprehensive Physiology, 2011 is used. This model assumes that the body is made up of four compartments perfused in parallel. The venous partial pressure of nitrogen leaving the $l$-th body compartment is found by solving the following conservation of mass equation :

$$\frac{dP_{vl}^{N_2}}{dt} = \frac{Q_T Q_l}{\lambda_l V_{tl}} \left( P_a^{N_2}(t) - P_{vl}^{N_2}(t) \right), \quad \text{(E67)}$$

where $P_{vl}^{N_2}(t)$ is the venous (which is assumed to be equal to the tissue) nitrogen partial pressure in body compartment $l$, $Q_l$ is the vascular conductance of body compartment $l$, $\lambda_l$ is the blood-tissue partition coefficient of compartment $l$, $V_{tl}$ is the

tissue volume of compartment $l$, and $P_a^{N_2}(t)$ is the perfusion weighted average arterial partial pressure of $N_2$ in each alveolar compartment. $P_a^{N2}(t)$ is given by

$$P_a^{N_2}(t) = \sum_i C_{d_i} F_i^{N_2}(t)(P_b - P_{H_2O}). \qquad (E68)$$

The mixed venous nitrogen concentration entering the lung is the perfusion weighted concentration leaving the $l$ body compartments,

$$C_{\bar{v}}^{N_2}(t) = S_b \sum_l Q_l P_{vl}^{N_2}(t) \qquad (E69)$$

where $S_b$ is the solubility of $N_2$ in the blood. An analogous approach may be taken with any tracer gases present.
Unlike $N_2$, $O_2$ is consumed by the body, and thus if the four compartment model for $O_2$ were adopted, how this consumption was distributed among the compartments would also need to be determined. As the information with which to do this is not available, for the $O_2$ stores a single body compartment is assumed. Further, a constant rate of $O_2$ consumption is assumed, $\dot{V}_{O_2}$, which is the same as that measured at the mouth during the air breathing period. Thus the governing differential equation for the high $O_2$ breathing period is

$$\frac{dC_{\bar{v}}^{O_2}}{dt} = \frac{1}{V_L} \left( Q_T \left( C_a^{O_2}(t) - C_{\bar{v}}^{O_2}(t) \right) - \dot{V}_{O_2} \right), \qquad (E70)$$

where $V_L$ is the volume of the compartment and $C_a^{O_2}(t)$ is the perfusion weighted average arterial concentration. $C_a^{O_2}$ is given by

$$C_a^{O_2}(t) = \sum_i C_{di} C_{ai}^{O_2}(t). \qquad (E71)$$

The $O_2$ consumption, $\dot{V}_{O_2}$, is assumed to be equal to that measured at the mouth during the air breathing period, and thus :

$$\dot{V}_{O_2} = \frac{1}{t_{air}} \int_0^{t_{air}} F_M^{O_2}(t)u(t)dt, \qquad (E72)$$

where $F_M^{O2}$ is the $O_2$ fraction measured at the mouth and $t_{air}$ is the duration of the air breathing period. This leaves one additional parameter, $V_L$, to be estimated during the parameter recovery procedure.

**Deadspace compartments**

In this section the governing advection equation for a deadspace compartment is derived. It is assumed that the deadspace compartment is a straight cylindrical pipe of radius R and length $L$. Also it is assumed that gravity can be neglected, and that the flow is:
incompressible; independent of $z$; axial; and independent of the polar co-ordinate θ (i.e. we assume axial symmetry).
Consider a region of axial length $dz$ as illustrated in Figure 8: The volume of gas $g$ in that region at time $t$ will be

$$AdzF^g(z,t), \qquad (E73)$$

where $F^g$ is the fraction of gas g and $A$ is the cross-sectional area of the cylinder. Since diffusion is ignored, the net volume of gas $g$ entering that region at time $t$ is,

$$u(t)(F^g(z,t) - F^g(z+dz,t)) \qquad (E74)$$

where $u(t)$ is the measured flow rate. By conservation of mass the change of volume of gas g in this region from $t$ to $t+dt$ is equal to the net volume of gas entering that region over the period and thus

$$Adz(F^g(z, t+dt) - F^g(z,t)) = \mathrm{d}tu(t)(F^g(z,t) - F^g(z+dz,t)) \quad \text{(E75)}$$

Dividing this expression by $dzdt$ and taking limits as these tend to zero, the governing advection equation for the deadspace compartments is:

$$\frac{\partial F^g(z,t)}{\partial t} = -\frac{u(t)}{A}\frac{\partial F^g(z,t)}{\partial z}. \qquad \text{(E76)}$$

**Parameter estimation**

In this section the minimisation problem that is solved to estimate the parameters of the model (listed in Table 1) from data collected during the washout procedure 100 is defined. For this the washout procedure 100 comprises ten minutes of air breathing followed by six minutes of breathing pure $O_2$.

The cost function, $R$, that is minimised to estimate the parameters of the model is

$$R = \sum_{\text{air-breathing}}((F_M^{CO_2}(t_l) - F_S^{CO_2}(t_l))^2 + (F_M^{O_2}(t_l) - F_S^{O_2}(t_l))^2 +$$

$$\sum_{\text{oxygen breathing}} u(t_l)^2(F_M^{N_2}(t_l) - F_S^{N_2}(t_l))^2, \qquad \text{(E77)}$$

where $F_M^g$ and $F_S^g$ are the measured and simulated fractions in the measurement plane of the MFS respectively. The 'air-breathing period' for the cost function above is defined as the five minutes of normal breathing immediately preceding the high $O_2$ breathing phase. The first two minutes of normal breathing are discarded to allow the subject to adjust to breathing on the MFS, and the following three minutes are used to allow the model to settle into a quasi steady state.

In (E77) the $CO_2$ and $O_2$ fluxes are considered during the air-breathing period, and the $N_2$ flux during the high O2 breathing period. The reason for not using $N_2$ during the air breathing period is that during this period the variation of $N_2$ is small, and thus the signal is relatively noisy.

During the $O_2$ breathing phase, we have only an approximate idea of how the venous $O_2$ and $CO_2$ concentrations behave. As the $N_2$ exchange with the blood is relatively limited, the influence of perfusion on its signal is much lower, and thus during the washout only the $N_2$ flux is considered.

To evaluate the cost function the forward problem must be solved, where the governing equations of the model (E56,E59,E60,E61,E62,E63,E65,E66,E67,E70,E76) are solved with known parameter values. However, when solving the forward problem, as well as values for the parameters and the inspired gas fractions and flows, it is also necessary to know:

a) initial conditions for the differential equations, and
b) the composition of the inspired gas and mixed venous blood.

The initial conditions required are the gas fractions in every deadspace and alveolar compartment, and the starting volumes of the alveolar compartments.

The mixed venous concentrations of $O_2$ and $CO_2$ may be estimated using a combination of the model, and data collected from the molecular flow sensor. Although highly damped, these venous concentrations are not entirely constant because volunteers may hyperventilate to some degree when breathing on the molecular flow sensor. For this reason a linear trend is allowed for during the air breathing period. During the air breathing period, a continuous flow version of the model in which all alveolar volumes are set to zero is used to provide initial estimates of the venous $CO_2$ and $O_2$ concentrations/partial pressures on every breath from the volumes of these gases exchanged at the mouth. The value of each of these is modelled as a constant value, plus a linear drift over time by:

1. Calculating the breath-by-breath $CO_2$ and $O_2$ exchange at the mouth directly from the experimental data.
2. Constructing the continuous flow analogue of the full respiratory model - this is a model in which all alveolar volumes are zero.
3. Using the continuous flow analogue model within a non-linear optimisation routine to estimate, for each breath separately, the mixed venous concentrations/ partial pressures and alveolar gas fractions that provide the experimentally measured values for that breath's CO2 and O2 exchange.

4. Employing linear regression to estimate the constant values and linear drift coefficients for the mixed venous values for $CO_2$ and $O_2$.

5. Setting the starting alveolar (and connected personal deadspace) gas fractions equal to the values calculated in step 3.

6. Setting the starting volumes of the alveolar compartments by: (i) estimating the difference between the total lung volume at the start of the first breath and FRC from the integral of the flow pattern; and (ii) adding this difference multiplied by the fractional compliance of the compartment to the compartments volume at FRC.

7. Running the full model within a non-linear optimisation routine, such as the function *fmincon* in Matlab (other non-linear optimisation routines may be used), to fine-tune the constant values for $CO_2$ and $O_2$ so as to minimise the squared deviations between experimental data and model output for the breath-to-breath gas exchange data.

Conversions between partial pressures and concentrations for $CO_2$ and $O_2$ in blood are obtained using a model of $CO_2$ and $O_2$ gas carriage by blood (such as Rees SE, Klæstrup E, Handy J, Andreassen S, Kristensen SR. "Mathematical modelling of the acid-base chemistry and oxygenation of blood: a mass balance, mass action approach including plasma and red blood cells." Eur J Appl Physiol 108: 483-494, 2010).

During the high $O_2$ breathing phase, the recirculating arterial $O_2$ will lead to a rise in the venous $O_2$ concentrations. A simple model of the body's oxygen stores with a single parameter, $V_L$ may be used. To estimate the behaviour of the mixed venous $O_2$ concentration during the high $O_2$ breathing period, a value for this parameter must be found. This is achieved by finding the value of $V_L$ that ensures the alveolar volume remains stable during the high $O_2$ breathing phase.

Since the venous concentrations are estimated with the model, they will vary as the parameters of the model change in each cost function evaluation. Thus every time the cost function is evaluated it is necessary to: establish initial conditions; estimate the venous gas concentrations during air breathing; find an appropriate value of $V_L$; and then simulate the full experimental procedure.

The non-linear parameter estimation routine requires initial guesses for all the parameters that are to be estimated. These are set to be standard values, such as those given in Table 2.

Once the non-linear estimation routine completes, the final set of lung parameter values which best fit the experimental data are output. This parameter set, together with dependent variables, provides the quantification of lung function.

Additionally, the pure shunt fraction for the lung (i.e. the difference between the measured and model-predicted $SpO_2$ values - which measures how much of the blood is not exposed to alveolar gas) may be calculated from the model output for pulmonary venous (systemic arterial) oxygen saturation, the experimentally recorded systemic arterial oxygen saturation and the systemic mixed venous (pulmonary arterial) oxygen saturation.

**Results**

Table 2 below gives values for the various parameters, derived values and observed quantities calculated by running the model for a healthy volunteer and which, where needed, may be used as initial conditions for running the optimisation routine for any other subject - the model parameters then being varied in the fitting process to match the particular subject's breathing.

Table 2

| Labels | Values | Descriptions |
|---|---|---|
| Patient sex | Male | |
| Patient weight/kg | 78 | |
| Patient height/cm | 177 | |
| | | |
| | | |
| **Lung parameters** | | |
| | | |
| FRC/l,BPTS | 4.725688443 | Functional residual capacity |
| Equivalent CO2 tissue volume | 0.25 | Effective tissue vol for CO2 (fraction of FRC) |

(continued)

| Lung parameters | | |
|---|---|---|
| DS/l,BTPS | 0.225313189 | Deadspace |
| sDS | 0.050065423 | Standardised deadspace (deadspace as fraction of FRC) |
| sigma(ln(Cp)) | 0.415668464 | Standard deviation of lung compliance distribution (log normal) |
| sigma(ln(vCd)) | 0.794680702 | Standard deviation of vascular conductance distribution (log normal) |
| rho(ln(Cp),ln(vCd)) | 0.8 | Correlation coefficient between log lung compliance and log vascular conductance distributions |
| sigma(ln(sDS) | 0.309420929 | Standard deviation of standardised deadspace distribution (normal) |
| sDs*sigma(ln(sDS) | 0.01549129 | Scaled deadspace distribution width |
| | | |
| | | |
| **Ventilation** | | |
| | | |
| VI/l/min,STPD | 10.76092456 | Inspired ventilation |
| VE/l/min,STPD | 10.72092393 | Expired ventilation |
| VA/l/min,STPD | 7.133845402 | Alveolar ventilation (inspired ventilaiton minus deadspace ventilation) |
| sigma(ln(VA)) | 0.56044194 | Standard deviation of alveolar ventilation distribution (log normal) |
| External DS/l | 0.155 | External deadspace (equipment) |
| | | |
| | | |
| **Blood flow** | | |
| | | |
| Q/l/min | 7.294108949 | Blood flow |
| CvCO2/ml STPD/100 ml | 49.52947517 | Systemic venous CO2 concentration |
| CvO2/ml STPD/100 ml | 15.34975624 | Systemic venous O2 concentration |
| CO2 Slope/ml STPD/100 ml min | -0.225976651 | Slope of systemic venous CO2 concentration |
| O2 Slope/ml STPD/100 ml min | 0.214332972 | Slope of systemic venous O2 concentration |
| CaCO2/ml STPD/100 ml | 44.94896812 | Systemic arterial CO2 concentration |
| CaO2/ml STPD/100 ml | 20.27702732 | Systemic arterial O2 concentration |
| SO2 air/% | 97.2 | Arterial O2 saturation - air breathing |
| SO2 air/% | 97.57861506 | Arterial O2 saturation - air breathing-model |
| .SO2 O2/% | 99 | Arterial O2 saturation - O2 breathing |
| SO2 O2/% | 99.93304663 | Arterial O2 saturation - O2 breathing-model |
| Shunt fraction/% | 0.390990771 | Shunt Fraction |
| V_L | 16.73387648 | Effective circulating volume of distribution for O2 |
| | | |

(continued)

| Blood flow | | |
|---|---|---|
| | | |
| **Gas exchange** | | |
| | | |
| VCO2/ml/min | 334.5746455 | CO2 production |
| VO2/ml/min | 364.7054474 | O2 consumption |
| VCO2 Efficiency | 96.49166712 | Efficiency of CO2 production: inhomogeneous versus homogenous lung |
| VO2 Efficiency | 97.93144679 | Efficiency of O2 consumption: inhomogenous versus homogenous lung |
| | | |
| | | |
| **Vdot/Q like parameters** | | |
| | | |
| VA_Q mean | 0.978028359 | Ratio of total alveolar ventilation to total blood flow |
| rho(ln(VA),ln(Q)) | 0.776251033 | Corrrelation coefficient between log alveolar ventilation and log blood flow distributions |
| Ln SD(ventilation) | 0.481803655 | Standard deviation of ventilation-perfusion ratio (ventilation) |
| Ln SD(perfusion) | 0.467905015 | Standard deviation of ventilation-perfusion ratio (perfusion) |
| Ln SD(volume) | 0.446734952 | Standard deviation of ventilation-perfusion ratio (volume) |
| RQ_mean | 0.917383187 | Respiratory quotient |
| sigma(RQ) | 0.315117454 | Standard deviation for respiratory quotient between lung units |
| | | |
| | | |
| **pCO2 pO2 (partial pressures) type parameters** | | |
| | | |
| PvCO2/mmHg | 48.40001075 | Systemic venous PCO2 |
| hPvCO2/mmHg | 46.63505599 | Systemic venous PCO2 for equivalent homogenous lung |
| (ih-h)PvCO2/mmHg | 1.764954763 | Difference in systemic venous PCO2 between inhomogenous and homogeneous lung |
| PETCO2/mmHg | 39.95967008 | End-tidal PCO2 |
| hPETCO2/mmHg | 39.32555271 | End-tidal PCO2 for equivalent homogenous lung |
| PACO2/mmHg | 39.13710995 | Mean alveolar PCO2 |
| sigma(PACO2)/mmHg | 3.906261331 | Standard deviation for alveolar PCO2 between lung units |
| PaCO2/mmHg | 40.89227222 | Systemic arterial PCO2 |
| (a-A)PCO2/mmHg | 1.755162266 | Systemic arterial to alveolar PCO2 difference |
| hPACO2/mmHg | 39.30217184 | Alveolar/ systemic arterial PCO2 for equivalent homogenous lung |
| (ih-h)PaCO2/mmHg | 1.590100376 | Difference in systemic arterial PCO2 between inhomogenous and homogenous lung |
| PvO2/mmHg | 41.15117784 | Systemic venous PO2 |
| hPvO2/mmHg | 41.13356798 | Systemic venous PO2 for equivalent homogenous lung |

(continued)

| pCO2 pO2 (partial pressures) type parameters | | |
|---|---|---|
| (ih-h)PvO2/mmHg | 0.017609866 | Difference in systemic venous PO2 between homogenous and inhomogenous lung |
| PETO2/mmHg | 107.5880803 | End-tidal PO2 |
| hPETO2/mmHg | 108.6012316 | End-tidal PO2 for equivalent homogenous lung |
| PAO2/mmHg | 108.8288646 | Mean alveolar PO2 |
| sigma(PAO2)/mmHg | 10.18524949 | Standard deviation for alveolar PO2 between lung units |
| PaO2/mmHg | 100.1778473 | Systemic arterial PCO2 |
| (a-A)PO2/mmHg | -8.651017259 | Systemic arterial to alveolar PCO2 difference |
| hPAO2/mmHg | 108.0606609 | Alveolar/ systemic arterial PO2 for equivalent homogenous lung |
| (ih-h)PaO2/mmHg | -7.882813554 | Difference in systemic arterial PO2 between homogenous and inhomogenous lung |

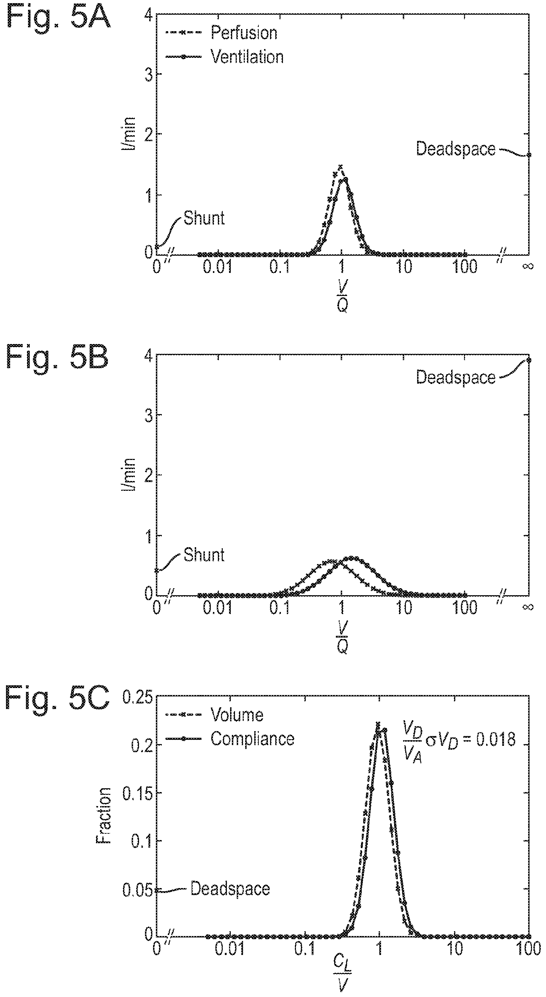

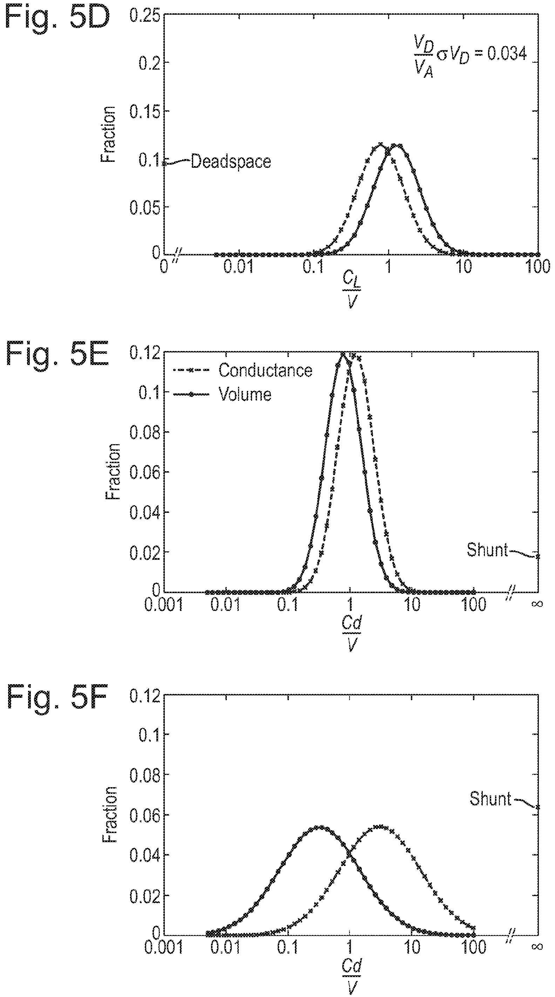

Figure 5 illustrates the results of performing the method above on a healthy volunteer (lefthand plots A, C and E) and on a volunteer with GOLD stage 2 COPD (right-hand plots B, D and F). Figures 5A and 5B show the distributions for ventilation:perfusion ratios ($\frac{\dot{V}}{\dot{V}}$) and it can be seen that the variance in Figure 5B for the COPD volunteer is much greater than for the heathy volunteer in Figure 5A. Figures 5C and D show the compliance:volume ratios ($\frac{C_L}{V}$) and Figures 5E and F show the vascular conductance:volume ratios ($\frac{C_D}{V}$) and again in both cases the variance for the COPD volunteer is much greater than for the healthy volunteer, thus illustrating a much greater heterogeneity (lower homogeneity) for the COPD volunteer's lung.

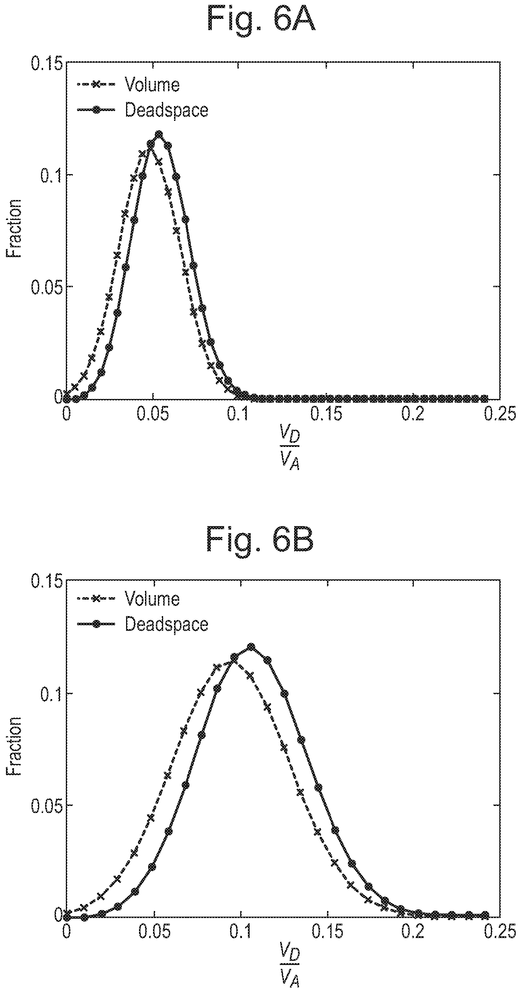

Figure 6A and B show the distributions for the deadspace:volume ratios ($\frac{V_D}{V_A}$) for a healthy volunteer (Figure 6A) and a COPD volunteer (Figure 6B), again showing the increase in variance with airway disease.

**Claims**

1. A method of quantifying lung function of a subject comprising:

   a step (100) of obtaining plural measurements of the amounts at the mouth of plural respiratory gases in inhaled and exhaled breath as a function of time within each breath of a plurality of successive breaths during a period of steady breathing and a period of inert gas wash-in or wash-out of a respiring subject (1);
   a step (200) of outputting the measured amounts to a data processor (30) adapted to model lung function by using a parameterised lung model adapted to predict expired amounts of the plural respiratory gases at the mouth of a respiring subject;
   a step (300) of varying the parameters of the lung model to fit the predicted expired respiratory gas amounts to the plural measurements; and
   a step (400) of outputting at least one parameter of the lung model as a quantifier of lung function
   **characterized in that**:

   the parameterised lung model is adapted to predict expired amounts of the plural respiratory gases at the mouth of a respiring subject at the time point of each of said plural measurements,
   the parameterized lung model models the lung as a plurality of alveolar compartments (VA1, VA2, VAi) connected by a respective plurality of personal deadspaces (VD1, VD2, VDi) to a common deadspace (VDC) leading to the mouth, and wherein the volumes of the personal deadspaces (VD1, VD2, VDi) are distributed with alveolar compartment volume (VA1, VA2, VAi) according to a personal deadspace distribution; and

the step (300) of varying the parameters of the lung model to fit the predicted expired respiratory gas amounts to the plural measurements comprises fitting the predicted expired respiratory gas amounts at each time point to the expiratory flow profile over each breath of the multiple successive breaths for one or more of the respiratory gases.

2. A method according to claim 1 wherein: a) the period of steady breathing and the period of inert gas wash-in or wash-out are simultaneous; or b) the period of steady breathing precedes the period of inert gas wash-in or wash-out; or c) periods of inert gas wash-in alternate with periods of inert gas wash-out.

3. A method according to claim 1 comprising estimating, from the measured amounts of respiratory gases, nitrogen wash-out for the respiring subject and varying the parameters of the lung model to fit the predicted expired amount of nitrogen during the wash-out to the estimated nitrogen wash-out.

4. A method according to any one of the preceding claims wherein the measurements of the amounts of respiratory gases comprise measurements of the molar flows, or the total flow with concentrations or fractions of respiratory gases in breath.

5. A method according to any one of the preceding claims wherein the measurements of the amounts of respiratory gases are made at least every 50ms, more preferably at least every 25ms, more preferably at least every 10ms.

6. A method according to any one of the preceding claims wherein the step (300) of varying the parameters of the lung model to fit the predicted expired respiratory gas amounts to the plural measurements comprises fitting the predicted expired carbon dioxide and oxygen amounts measured during the period of steady breathing and fitting the inert gas amounts in the period of inert gas wash-in or wash-out.

7. A method according to any preceding claim wherein the parametrized model comprises: the volume of the common deadspace ($V_{DC}$); the fractional volume of each alveolar compartment ($V_{A1}$, $V_{A2}$, $V_{Ai}$), being the fraction of the total alveolar volume of each compartment at the functional residual capacity of the model; the fractional expansion or compliance of each alveolar compartment, being approximately the fraction of the flow measured at the mouth received by each alveolar compartment; the volume of the personal deadspace ($V_{D1}$, $V_{D2}$, $V_{Di}$) for each alveolar compartment; the vascular conductance of each alveolar compartment, being the fraction of the total perfusion received by each compartment.

8. A method according to any preceding claim wherein the model parameters define at least one of: a) a bivariate lognormal distribution for the variation of fractional lung compliance with fractional volume and the variation of vascular conductance with fractional volume, the bivariate lognormal distribution being defined by the variance of the log of the fractional lung compliance with fractional volume distribution, the variance of the log of the vascular conductance with fractional volume distribution, and their correlation; and b) a normal, or lognormal, distribution for the variation of the fractional personal deadspace volume with the fractional compartment volume.

9. A method according to any one of the preceding claims wherein measurements of the inspired amounts of respiratory gases are input to the parametrized model.

10. An apparatus for quantifying lung impairment in accordance with the method of any one of the preceding claims and comprising:

a molecular flow sensor (10) for obtaining measurements of the amounts at the mouth of plural respiratory gases in inhaled and exhaled breath as a function of time within each breath of a plurality of successive breaths during the period of steady breathing and the period of inert gas wash-in or wash-out of a respiring subject (1);
a data processor (30)

wherein the said apparatus is configured to execute the method of any of the preceding claims.

11. Apparatus according to claim 10 wherein the molecular flow sensor (10) is adapted to measure the amounts of oxygen and carbon dioxide in an airway at the mouth of the respiring subject (1).

12. Apparatus according to claim 10 or 11 wherein the molecular flow sensor (10) is adapted to measure the molar flows, or the total flow with concentrations or fractions of respiratory gases in the breath of a respiring subject.

13. Apparatus according to claim 10, 11, or 12 wherein the molecular flow sensor (10) is adapted to measure the amounts of respiratory gases at least every 50ms, more preferably at least every 25ms, more preferably at least every 10ms.

14. A computer program comprising program code means for controlling a computer to execute the method of any one of the claims 1 to 9.

**Patentansprüche**

1. Verfahren zur Quantifizierung der Lungenfunktion eines Patienten, das umfasst:

   einen Schritt (100) zum Erhalten mehrerer Messungen der Mengen mehrerer Atemgase in eingeatmeter und ausgeatmeter Luft am Mund als Funktion der Zeit innerhalb jedes Atemzugs einer Vielzahl aufeinanderfolgender Atemzüge während einer Periode gleichmäßiger Atmung und einer Periode des Ein- oder Auswaschens von Inertgas eines atmenden Patienten (I);
   einen Schritt (200) zum Ausgeben der gemessenen Mengen an einen Datenprozessor (30), der zum Modellieren der Lungenfunktion unter Verwendung eines parametrisierten Lungenmodells ausgelegt ist, das zum Vorhersagen der ausgeatmeten Mengen der mehreren Atemgase am Mund eines atmenden Patienten ausgelegt ist;
   einen Schritt (300) zum Variieren der Parameter des Lungenmodells, um die vorhergesagten ausgeatmeten Atemgasmengen an die mehreren Messungen anzupassen; und
   einen Schritt (400) zum Ausgeben mindestens eines Parameters des Lungenmodells als Quantifizierer der Lungenfunktion,
   **dadurch gekennzeichnet, dass**
   das parametrisierte Lungenmodell dafür ausgelegt ist, die ausgeatmeten Mengen der mehreren Atemgase am Mund eines atmenden Patienten zum Zeitpunkt jeder der mehreren Messungen vorherzusagen,
   das parametrisierte Lungenmodell die Lunge als eine Vielzahl von Alveolarkompartimenten (VA1, VA2, VAi) modelliert, die durch eine jeweilige Vielzahl von persönlichen Toträumen (VD1, VD2, VDi) mit einem gemeinsamen Totraum (VDC) verbunden sind, der zum Mund führt, und wobei die Volumina der persönlichen Toträume (VD1, VD2, VDi) mit dem Volumen der Alveolarkompartimente (VA1, VA2, VAi) gemäß einer persönlichen Totraumverteilung verteilt sind; und
   der Schritt (300) zum Variieren der Parameter des Lungenmodells, um die vorhergesagten ausgeatmeten Atemgasmengen an die mehreren Messungen anzupassen, das Anpassen der vorhergesagten ausgeatmeten Atemgasmengen zu jedem Zeitpunkt an das Ausatmungsstromprofil über jeden Atemzug der mehreren aufeinanderfolgenden Atemzüge für eines oder mehrere der Atemgase umfasst.

2. Verfahren nach Anspruch 1, wobei: a) die Periode der gleichmäßigen Atmung und die Periode des Einwaschens oder Auswaschens des Inertgases gleichzeitig sind; oder b) die Periode der gleichmäßigen Atmung der Periode des Einwaschens oder Auswaschens des Inertgases vorausgeht; oder c) Perioden des Einwaschens des Inertgases sich mit Perioden des Auswaschens des Inertgases abwechseln.

3. Verfahren nach Anspruch 1, das Folgendes umfasst: Schätzen des Stickstoffauswaschens für den atmenden Patienten aus den gemessenen Mengen der Atemgase und Variieren der Parameter des Lungenmodells, um die vorhergesagte ausgeatmete Stickstoffmenge während des Auswaschens an das geschätzte Stickstoffauswaschen anzupassen.

4. Verfahren nach einem der vorhergehenden Ansprüche, wobei die Messungen der Mengen an Atemgasen Messungen der molaren Ströme oder des Gesamtstroms mit Konzentrationen oder Anteilen von Atemgasen in der Atemluft umfassen.

5. Verfahren nach einem der vorhergehenden Ansprüche, wobei die Messungen der Mengen an Atemgasen mindestens alle 50 ms, vorzugsweise mindestens alle 25 ms, noch bevorzugter mindestens alle 10 ms erfolgen.

6. Verfahren nach einem der vorhergehenden Ansprüche, wobei der Schritt (300) zum Variieren der Parameter des Lungenmodells, um die vorhergesagten ausgeatmeten Atemgasmengen an die mehreren Messungen anzupassen, das Anpassen der vorhergesagten ausgeatmeten Kohlendioxid- und Sauerstoffmengen, die während der Phase der gleichmäßigen Atmung gemessen wurden, und Anpassen der Inertgasmengen in der Phase des Einwaschens oder Auswaschens von Inertgas umfasst.

7. Verfahren nach einem der vorhergehenden Ansprüche, wobei das parametrisierte Modell umfasst: das Volumen des gemeinsamen Totraums ($V_{DC}$); das Teilvolumen jedes Alveolarkompartiments ($V_{A1}$, $V_{A2}$, $V_{Ai}$), das der Anteil des gesamten Alveolarvolumens jedes Kompartiments bei der funktionellen Residualkapazität des Modells ist; die fraktionelle Expansion oder Compliance jedes Alveolarkompartiments, die etwa dem Anteil des am Mund gemessenen Stroms entspricht, der von jedem Alveolarkompartiment aufgenommen wird; das Volumen des persönlichen Totraums ($V_{D1}$, $V_{D2}$, $V_{Di}$) für jedes Alveolarkompartiment; die Gefäßleitfähigkeit jedes Alveolarkompartiments, die dem Anteil der Gesamtperfusion entspricht, die jedes Kompartiment erhält.

8. Verfahren nach einem der vorhergehenden Ansprüche, wobei die Modellparameter mindestens eines der Folgenden definieren: a) eine bivariate lognormale Verteilung für die Variation der fraktionellen Lungencompliance mit dem fraktionellen Volumen und die Variation der Gefäßleitfähigkeit mit dem fraktionellen Volumen, wobei die bivariate lognormale Verteilung durch die Varianz des Logarithmus der fraktionellen Lungencompliance mit der fraktionellen Volumenverteilung, die Varianz des Logarithmus der Gefäßleitfähigkeit mit der fraktionellen Volumenverteilung und deren Korrelation definiert ist; und b) eine normale oder lognormale Verteilung für die Variation des fraktionellen persönlichen Totraumvolumens mit dem fraktionellen Kompartimentvolumen.

9. Verfahren nach einem der vorhergehenden Ansprüche, wobei Messungen der eingeatmeten Mengen von Atemgasen in das parametrisierte Modell eingegeben werden.

10. Vorrichtung zur Quantifizierung einer Lungenfunktionsstörung nach einem der vorhergehenden Ansprüche, die umfasst:

    einen Molekularstromsensor (10) zum Erhalten von Messungen der Mengen mehrerer Atemgase in eingeatmetem und ausgeatmetem Atem an der Mundöffnung als Funktion der Zeit innerhalb jedes Atemzugs einer Vielzahl aufeinanderfolgender Atemzüge während der Periode der gleichmäßigen Atmung und der Periode des Ein- oder Auswaschens von Inertgas eines atmenden Patienten (1);
    einen Datenprozessor (30)
    wobei die Vorrichtung dafür konfiguriert ist, das Verfahren nach einem der vorhergehenden Ansprüche auszuführen.

11. Vorrichtung nach Anspruch 10, wobei der Molekularstromsensor (10) dafür ausgelegt ist, die Mengen an Sauerstoff und Kohlendioxid in einem Atemweg an der Mundöffnung des atmenden Patienten (1) zu messen.

12. Vorrichtung nach Anspruch 10 oder 11, wobei der Molekularstromsensor (10) dafür ausgelegt ist, die Molströme oder den Gesamtstrom mit Konzentrationen oder Anteilen von Atemgasen in der Atmung eines atmenden Patienten zu messen.

13. Vorrichtung nach Anspruch 10, 11 oder 12, wobei der Molekularstromsensor (10) dafür ausgelegt ist, die Mengen an Atemgasen mindestens alle 50 ms, vorzugsweise mindestens alle 25 ms, noch bevorzugter mindestens alle 10 ms zu messen.

14. Computerprogramm, das Programmcodemittel zum Steuern eines Computers umfasst, um das Verfahren nach einem der Ansprüche 1 bis 9 auszuführen.

**Revendications**

1. Procédé de la quantification de fonction pulmonaire d'un sujet, comprenant :

    une étape (100) consistant à obtenir une pluralité de mesures des quantités, au niveau de la bouche, d'une pluralité de gaz respiratoires dans une respiration inhalée et exhalée en fonction du temps dans les limites de chaque respiration d'une pluralité de respirations successives durant une période de respiration stable et une période d'inspiration ou d'expiration de gaz inerte d'un sujet respirant (1) ;
    une étape (200) consistant à envoyer les quantités mesurées à un processeur de données (30) adapté pour modéliser une fonction pulmonaire en utilisant un modèle pulmonaire paramétré adapté pour prédire des quantités expirées de la pluralité de gaz respiratoires au niveau de la bouche d'un sujet respirant ;
    une étape (300) consistant à varier les paramètres du modèle pulmonaire pour adapter les quantités de gaz respiratoires expirées prédites à la pluralité de mesures ; et

une étape (400) consistant à sortir au moins un paramètre du modèle pulmonaire en tant que quantificateur de fonction pulmonaire
**caractérisé en ce que** :

le modèle pulmonaire paramétré est adapté pour prédire des quantités expirées de la pluralité de gaz respiratoires au niveau de la bouche d'un suj et respirant à l'instant de chacune desdites pluralité de mesures, le modèle pulmonaire paramétré modélise le poumon sous forme d'une pluralité de compartiments alvéolaires (VA1, VA2, VAi) raccordés par une pluralité respective d'espaces morts personnels (VD1, VD2, VDi) à un espace mort commun (VDC) menant à la bouche, et dans lequel les volumes des espaces morts personnels (VD1, VD2, VDi) sont distribués avec un volume de compartiments alvéolaires (VA1, VA2, VAi) selon une distribution d'espaces morts personnels ; et
l'étape (300) consistant à varier les paramètres du modèle pulmonaire pour adapter les quantités de gaz respiratoires expirées prédites à la pluralité de mesures comprend le fait d'adapter les quantités de gaz respiratoires expirées prédites à chaque instant au profil de débit expiratoire durant chaque respiration des multiples respirations successives pour un ou plusieurs des gaz respiratoires.

2.  Procédé selon la revendication 1, dans lequel : a) la période de respiration stable et la période d'inspiration ou d'expiration de gaz inerte sont simultanées ; ou b) la période de respiration stable précède la période d'inspiration ou d'expiration de gaz inerte ; ou c) des périodes d'inspiration de gaz inerte s'alternent avec des périodes d'expiration de gaz inerte.

3.  Procédé selon la revendication 1, comprenant l'estimation, à partir des quantités mesurées de gaz respiratoires, d'expiration d'azote pour le sujet respirant et la variation des paramètres du modèle pulmonaire pour adapter la quantité expirée prédite d'azote durant l'expiration à l'expiration d'azote estimée.

4.  Procédé selon l'une quelconque des revendications précédentes, dans lequel les mesures des quantités de gaz respiratoires comprennent des mesures des débits molaires, ou du débit total avec des concentrations ou fractions de gaz respiratoires dans la respiration.

5.  Procédé selon l'une quelconque des revendications précédentes, dans lequel les mesures des quantités de gaz respiratoires sont effectuées au moins toutes les 50 ms, de façon davantage préférée au moins toutes les 25 ms, de façon davantage préférée au moins toutes les 10 ms.

6.  Procédé selon l'une quelconque des revendications précédentes, dans lequel l'étape (300) consistant à varier les paramètres du modèle pulmonaire pour adapter les quantités de gaz respiratoires expirées prédites à la pluralité de mesures comprend l'adaptation des quantités expirées prédites de dioxyde de carbone et d'oxygène mesurées durant la période de respiration stable et l'adaptation des quantité de gaz inerte dans la période d'inspiration ou d'expiration de gaz inerte.

7.  Procédé selon une quelconque revendication précédente, dans lequel le modèle paramétré comprend : le volume de l'espace mort commun ($V_{DC}$) ; le volume fractionnel de chaque compartiment alvéolaire ($V_{A1}$, $V_{A2}$, $V_{Ai}$), étant la fraction du volume alvéolaire total de chaque compartiment à la capacité résiduelle fonctionnelle du modèle ; l'expansion ou compliance fractionnelle de chaque compartiment alvéolaire, étant approximativement la fraction du débit mesuré au niveau de la bouche reçu par chaque compartiment alvéolaire ; le volume de l'espace mort personnel ($V_{D1}$, $V_{D2}$, $V_{Di}$) pour chaque compartiment alvéolaire ; la conductance vasculaire de chaque compartiment alvéolaire, étant la fraction de la perfusion totale reçue par chaque compartiment.

8.  Procédé selon une quelconque revendication précédente, dans lequel les paramètres de modèle définissent au moins une de : a) une distribution normale logarithmique à deux variables pour la variation de compliance pulmonaire fractionnelle avec volume fractionnel et la variation de conductance vasculaire avec volume fractionnel, la distribution normale logarithmique à deux variables étant définie par la variance du logarithme de la compliance pulmonaire fractionnelle avec distribution de volume fractionnel, la variance du logarithme de la conductance vasculaire avec la distribution de volume fractionnel, et leur corrélation ; et b) une distribution normale, ou normale logarithmique, pour la variation du volume d'espace mort personnel fractionnel avec le volume compartiment fractionnel.

9.  Procédé selon l'une quelconque des revendications précédentes, dans lequel des mesures des quantités inspirées de gaz respiratoires sont entrées dans le modèle paramétré.

**10.** Appareil pour la quantification de déficience pulmonaire conformément au procédé de l'une quelconque des revendications précédentes, et comprenant :

un capteur de débit moléculaire (10) destiné à obtenir des mesures des quantités au niveau de la bouche de pluralité de gaz respiratoires dans une respiration inhalée et exhalée en fonction du temps dans les limites de chaque respiration d'une pluralité de respirations successives durant la période de respiration stable et la période d'inspiration ou d'expiration de gaz inerte d'un sujet respirant (1) ;
un processeur de données (30),
dans lequel ledit appareil est configuré pour exécuter le procédé de quelconques des revendications précédentes.

**11.** Appareil selon la revendication 10 dans lequel le capteur de débit moléculaire (10) est adapté pour mesurer les quantités d'oxygène et de dioxyde de carbone dans des voies respiratoires au niveau de la bouche du sujet respirant (1).

**12.** Appareil selon la revendication 10 ou 11, dans lequel le capteur de débit moléculaire (10) est adapté pour mesurer les débits molaires, ou le débit total avec des concentrations ou fractions de gaz respiratoires dans la respiration d'un sujet respirant.

**13.** Appareil selon la revendication 10, 11, ou 12, dans lequel le capteur de débit moléculaire (10) est adapté pour mesurer les quantités de gaz respiratoires au moins toutes les 50 ms, de façon davantage préférée au moins toutes les 25 ms, de façon davantage préférée au moins toutes les 10 ms.

**14.** Programme d'ordinateur, comprenant des moyens de code de programme pour commander un ordinateur pour exécuter le procédé de l'une quelconque des revendications 1 à 9.

# Fig. 1

Collect Nitrogen washout data using
molecular flow sensor — 100

Use measured breathing pattern
and inspired gas fraction

Simulate procedure using lung model — 200

Compare mass fluxes of gases in
10ms periods

Check correspondence between
simulation and data — 300

Vary parameters until
the closest fit between
simulated and
experimental data
is found

Report outputs of the lung model as
indices for lung function — 400

# Fig. 2

Breath In/Out

# Fig. 3

# Fig. 4

Fig. 5A

Fig. 5B

Fig. 5C

Fig. 5D

Fig. 5E

Fig. 5F

## Fig. 6A

## Fig. 6B

# Fig. 7

# Fig. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150272475 A1

**Non-patent literature cited in the description**

- **KENNETH C BECK et al.** Ventilation-Perfusion Distribution in Normal Subjects. *J. Appl. Physiol.*, 2012, vol. 113, 872-877
- **GUSTAFSSON et al.** Slow and Fast Lung Compartments in Cystic Fibrosis Measured by Nitrogen Multiple-Breath Wash-out. *J. Appl. Physiol.*, 2014, vol. 117, 720-729
- **P D ROBINSON et al.** *European Respiratory Journal*, 2013, vol. 41, 507-522
- **LUCA CIAFFONI et al.** In-airway molecular flow sensing: A new technology for continuous, non-invasive monitoring of oxygen consumption in critical care. *Science Advances*, 10 August 2016, vol. 2 (8), e1600560-e1600560
- **CIAFFONI, L.** ; **O'NEILL, D. P.** ; **COUPER, J. H.** ; **RITCHIE, G. A.** ; **HANCOCK, G.** ; **ROBBINS, P. A.** In-airway molecular flow sensing: A new technology for continuous, non-invasive monitoring of oxygen consumption in critical care. *Science Advances*, 2016, vol. 2 (8), e1600560
- **WILSON**. *JAP*, 1992, vol. 72, 2298-2304
- **BECK**. *JAP*, 2001, vol. 90, 2151-2156
- **BECK**. *JAP*, 2012, vol. 113, 872-877
- **CANDER**. *JAP*, 1959, vol. 14, 541-551
- **SACKNER**. *JAP*, 1964, vol. 19, 374-380
- **C. B. DO**. *The multivariate Gaussian distribution*
- **REES SE** ; **KLÆSTRUP E** ; **HANDY J** ; **ANDREASSEN S** ; **KRISTENSEN SR**. Mathematical modelling of the acid-base chemistry and oxygenation of blood: a mass balance, mass action approach including plasma and red blood cells. *Eur J Appl Physiol*, 2010, vol. 108, 483-494